(19) Europäisches Patentamt
European Patent Office
Office européen des brevets

(11) Veröffentlichungsnummer: **0 189 374 B1**

# (12) EUROPÄISCHE PATENTSCHRIFT

(45) Veröffentlichungstag der Patentschrift: **18.03.92**

(51) Int. Cl.$^5$: **C07D 249/20**, C08K 5/34

(21) Anmeldenummer: **86810020.7**

(22) Anmeldetag: **17.01.86**

Die Akte enthält technische Angaben, die nach dem Eingang der Anmeldung eingereicht wurden und die nicht in dieser Patentschrift enthalten sind.

## (54) Flüssige 2-(2-Hydroxy-3-höherverzweigtalkyl-5-methylphenyl)-2H-benztriazolgemische, Verfahren zu deren Herstellung und diese enthaltende stabilisierte Zusammensetzungen.

(30) Priorität: **22.01.85 US 693483**

(43) Veröffentlichungstag der Anmeldung:
**30.07.86 Patentblatt 86/31**

(45) Bekanntmachung des Hinweises auf die Patenterteilung:
**18.03.92 Patentblatt 92/12**

(84) Benannte Vertragsstaaten:
**BE DE FR GB IT NL**

(56) Entgegenhaltungen:
**DE-A- 2 130 322**
**FR-A- 1 356 243**
**US-A- 3 983 132**

(73) Patentinhaber: **CIBA-GEIGY AG**
**Klybeckstrasse 141**
**CH-4002 Basel(CH)**

(72) Erfinder: **Winter, Roland A. E.**
**23 Banksville Road**
**Armonk New York 10504(US)**
Erfinder: **Detlefsen, Robert E.**
**Route 3 Box 256 Somerset Lane**
**Putnam Valley New York 10579(US)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Vorliegende Erfindung betrifft ausgewählte flüssige 2-Aryl-2H-benztriazolgemische zur Verwendung beim Schutz lichtempfindlicher organischer Materialien gegen Abbau, Verfahren zur Herstellung dieser Gemische und letztere enthaltende stabilisierte Zusammensetzungen.

UV-Absorber aus der o-Hydroxyphenyl-2H-benztriazolklasse sind seit langem als wirksame Lichtschutzmittel für organische Materialien bekannt und im Handel gut eingeführt.

Die Beschreibung, Herstellung und Anwendungen dieser wertvollen 2-Aryl-2H-Benztriazole sind im einzelnen in U.S. Patenten 3 004 896, 3 055 896, 3 072 585, 3 074 910, 3 189 615 und 3 230 194 zu finden.

Die bisher bekannten 2-Aryl-2H-benztriazole aus dieser Gruppe besitzen jedoch unter Umständen nur eine begrenzte Verträglichkeit in gewissen Substraten sowie eine übermässige Tendenz zum Ausschwitzen, Sublimieren und/oder Verflüchtigen bei der Verarbeitung stabilisierter Zusammensetzungen zu Platten, Folien, Fasern oder anderen flächigen Gebilden, wenn die Verarbeitung bei erhöhten Temperaturen erfolgen muss. Ferner können solche Benztriazole zu hohe Verluste durch Verflüchtigung oder Sublimation aus fertiggestellten Gebilden erleiden, insbesondere dünnen Folien oder Ueberzügen, vor allem, wenn sie im Gebrauch erhöhten Temperaturen ausgesetzt sind.

Es wurde versucht, durch Abänderung der Struktur der Benztriazole die Verträglichkeit zu erhöhen und den Verflüchtigungsverlust zu verringern.

Im U.S. Patent 3 230 194 wurde das Methyl durch eine höhere Alkylgruppe ersetzt, und die so erhaltene Verbindung 2-(2-Hydroxy-5-tert.-octylphenyl)-2H-benztriazol wies eine überlegene Verträglichkeit und Leistung in Polyäthylen gegenüber der obigen Verbindung auf.

U.S. Patente 4 283 327, 4 278 590 und 4 383 863 beschreiben 2-(2-Hydroxy-3,5-di-tert.-octylphenyl)-2H-benztriazol, das eine hervorragende Kombination von Verträglichkeit und/oder Löslichkeit in zahlreichen polymeren Substraten zusammen mit überlegener Beständigkeit gegen Verlust aus stabilisierten Zusammensetzungen bei der Hochtemperaturverarbeitung oder in Endverbrauchsanwendungen besitzt, wo Ueberzüge oder Folien aus den stabilisierten Zusammensetzungen selbst normalen Wettereinflüssen und Lichteinwirkungen ausgesetzt sind, und in photographischen Anwendungen. 2-(2-Hydroxy-3,5-di-tert.-octylphenyl)-2H-benztriazol ist jedoch immer noch ein Feststoff (Schmelzpunkt 105-106 °C), der bei zahlreichen Endverbrauchsanwendungen den gleichzeitigen Einsatz eines Lösungsmittels oder dispergierenden Verdünnungsmittels erfordert, damit es praktisch verwendbar wird. Solche Lösungs- oder Verdünnungsmittel sind aber aus Kosten- und Umweltgründen sowie unter weiteren Gesichtspunkten unerwünscht.

U.S. Patente 3 983 132, 4 096 242 und 4 129 521 beschreiben flüssige Gemische aus 2-(2-Hydroxy-5-nonylphenyl)-2H-benztriazolen bzw. 2-(2-Hydroxy-5-dodecylphenyl)-2H-benztriazolen sowie stabilisierte Zusammensetzungen unter Verwendung dieser Gemische, wobei die Nonyl- bzw. Dodecylgruppen jeweils ein isomeres Gemisch sekundärer und tertiärer, in para-Stellung zur Hydroxylgruppe an den 2-Phenylteil im 2H-Benztriazol gebundener Nonyl- bzw. Dodecylgruppen darstellt. Dabei werden die isomeren Nonyl- bzw. Dodecylgruppen in das Phenol eingeführt, bevor dieses nach der klassischen 2H-Benztriazolsynthese mit dem 2-Nitrophenyldiazoniumsalz gekuppelt wird.

Die vorliegenden flüssigen Benztriazole unterscheiden sich von denen aus diesen drei Patenten durch ihre Herstellungsmethode, durch die ortho-Stellung der verzweigten Alkylgruppe zur Hydroxylgruppe und teilweise, im Fall der Herstellung aus einem geradkettigen Alken, durch die Art der verzweigten Alkylgruppe selbst.

Die gemäss U.S. Patent 4 129 521 hergestellten flüssigen Gemische tragen keinen Substituenten in der ortho-Stellung zur Hydroxylgruppe, wodurch diese Verbindungen gegen Wechselwirkungen mit Metallionen während der Harzhärtung und bei anderen Endverbrauchsanwendungen in polymeren Substraten anfällig werden, was zu schädlichen Auswirkungen auf die Farbe, Lichtstabilität und Begleiteigenschaften führen kann. Die vorliegenden Gemische sind in der ortho-Stellung zur Hydroxylgruppe substituiert, und dieses Problem entfällt.

Gewisse hydrophobe, nicht-diffundierende Hydroxyphenylbenztriazole wurden als sehr nützliche Ultraviolettlichtabsorber in photographischen Gelatineschichten beschrieben (U.S. Patent 3 253 921). Wegen ihrer flüssigen oder nicht-kristallinen Art, ihren erwünschten Absorptionseigenschaften im ultravioletten Bereich und ihrer photographischen Reaktionsunfähigkeit sind die vorliegenden Benztriazole in photographischen Zusammensetzungen besonders nützlich, vor allem zum Schutz von Farbstoffbildern gegen die schädlichen Wirkungen ultravioletten Lichts.

U.S. Patent 3 253 921 beschreibt Benztriazole in weitem Sinn, gibt jedoch keine Beispiele für die vorliegenden Benztriazole, die bei der Stabilisierung photographischer Zusammensetzungen gegen die schädlichen Wirkungen ultravioletter Strahlung besonders wirksam sind.

Der Stand der Technik im Gebiet der Stabilisierung photographischer Farbstoffbilder wird ferner durch U.S. Patent 4 042 394 beleuchtet, wo die verschiedenen Komponenten in photographischen Zusammensetzungen und die Erfordernisse zur Stabilisierung photographischer Farbstoffbilder beschrieben sind.

U.S. Patente 4 383 863 und 4 447 511 erläutern die Verwendung von 2-(2-Hydroxy-3,5-di-tert.-octylphenyl)-2H-benztriazol in photographischen Elementen und Zusammensetzungen. Dieses bestimmte Benztriazol weist zwar erhöhte Löslichkeit in den verschiedenen für photographische Elemente verwendeten Lösungsmittein und Verdünnungsmitteln auf, doch sind solche Lösungs- und Verdünnungsmittel trotzdem erforderlich, da dieses Benztriazol immer noch ein kristalliner Feststoff ist.

In der deutschen Offenlegungsschrift 2 130 322 sind ebenfalls Lichtschutzmittel der 2-(2-Hydroxyphenyl)benztriazolreihe beschrieben, die in ortho-Stellung zur OH-Gruppe einen sekundären Alkylrest tragen und in para-Stellung durch Alkyl (z.B. Methyl) substituiert sind, sowie Mischungen dieser Verbindungen. Diese Verbindungen bzw. Mischungen werden aber ebenfalls nach der konventionellen Herstellungsmethode erhalten, wie oben bei der Besprechung von z.B. des US-Patentes 4 129 521 beschrieben. Die vorliegenden durch Alkylierung des Hydroxyphenylbenztriazolgerüstes selbst erhältlichen Mischungen unterscheiden sich von denen der DE-OS 2 130 322 in der Zusammensetzung und weisen überraschenderweise auch bessere Eigenschaften hinsichtlich ihrer Brauchbarkeit als UV-Absorber auf.

Die französische Patentschrift 1 356 243 betrifft photographische Zusammensetzungen, die als UV-Absorber ein 2-(2-Hydroxyphenyl)benztriazol enthalten. Es sind darin keine Mischungen von solchen Verbindungen untereinander beschrieben.

Die vorliegenden Benztriazolgemische sind flüssig oder nicht-kristallin, so dass wenig oder gar kein Lösungs- oder Verdünnungsmittel erforderlich ist und dünnere photographische Schichten mit allen daraus entstehenden wirtschaftlichen Vorteilen erhältlich sind.

Gegenstand vorliegender Erfindung sind ausgewählte flüssige oder nicht-kristalline 2-Aryl-2H-benztriazollichtabsorptionsmittel und damit stabilisierte, sowohl polymere als auch nicht-polymere organische Materialien, sowie diese flüssigen Materialien enthaltende photographische Elemente. Zu den stabilisierten Zusammensetzungen zählen Kunststoffe, Ueberzüge, Fasern, Folien und photographische Substrate.

Ein weiterer Gegenstand dieser Erfindung ist das Verfahren zur Herstellung dieser flüssigen oder nichtkristallinen Benztriazolgemische. Diese zeigen hohe Beständigkeit gegen Verflüchtigung, erhöhte Löslichkeit in ausgewählten Lösungsmitteln, erwünschte Absorptionseigenschaften im ultravioletten Bereich und photographische Reaktionsunfähigkeit. Dank dieser Eigenschaftskombination sind diese Benztriazole besonders in photographischen Zusammensetzungen nützlich, vor allem zum Schutz von Farbstoffbildern gegen die schädlichen Einwirkungen ultravioletten Lichts.

Gegenstand vorliegender Erfindung im einzelnen sind flüssige oder nicht-kristalline Gemische von Benztriazolen, die im wesentlichen aus Verbindungen der Formel

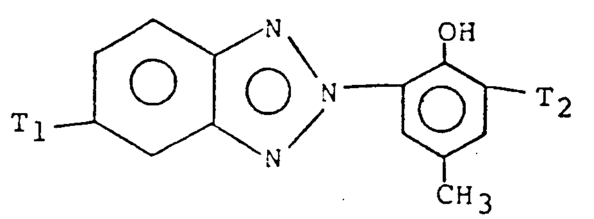

(I)

bestehen, worin $T_1$ Wasserstoff oder Chlor und $T_2$ ein statistisches Gemisch aus mindestens drei isomeren verzweigten sekundären Alkylgruppen mit je 8 bis 30 Kohlenstoffatomen der Formel

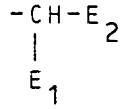

worin $E_1$ für geradkettiges Alkyl mit 1 bis 14 Kohlenstoffatomen und $E_2$ für geradkettiges Alkyl mit 4 bis 15 Kohlenstoffatomen stehen, wobei die Gesamtzahl Kohlenstoffatome in $E_1$ plus $E_2$ 7 bis 29 beträgt, darstellen, erhältlich durch Alkylierung einer Verbindung der Formel

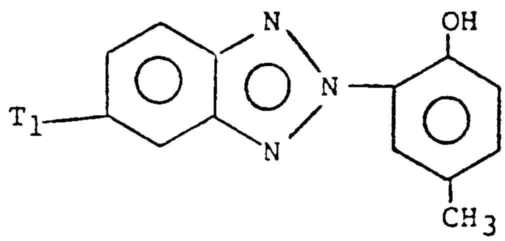

mit einem geradkettigen Alken mit 8 bis 30 Kohlenstoffatomen in Gegenwart eines sauren Katalysators und bei einer Temperatur von 100-200 ° C.

$T_1$ steht bevorzugt für Wasserstoff.

$T_2$ bedeutet vorzugsweise Alkyl mit 8 bis 16 Kohlenstoffatomen und besonders bevorzugt Alkyl mit 10 bis 12 Kohlenstoffatomen.

Diese bevorzugten und besonders bevorzugten Werte für den Kohlenstoffatomgehalt in $T_2$ bestimmen ebenfalls die bevorzugten und besonders bevorzugten Werte für $E_1$ und $E_2$, deren Kohlenstoffatomsumme eins weniger als die Summe für $T_2$ beträgt.

Gegenstand einer anderen Ausführungsform der vorliegenden Erfindung sind flüssige oder nichtkristalline Gemische von Benztriazolen, die im wesentlichen aus Verbindungen der Formel

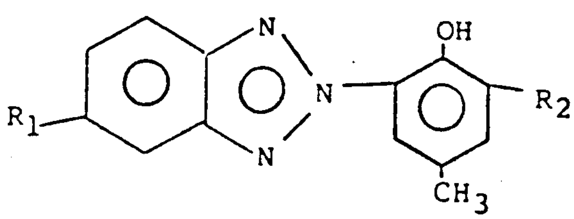

(II)

bestehen, worin $R_1$ Wasserstoff oder Chlor und $R_2$ ein statistisches Gemisch aus mindestens drei isomeren verzweigten Alkylgruppen mit je 8 bis 30 Kohlenstoffatomen und mit einer Mehrzahl Alkylverzweigungen entlang der Hauptalkylkette darstellen, erhältlich durch Alkylierung eines Benztriazols der Formel

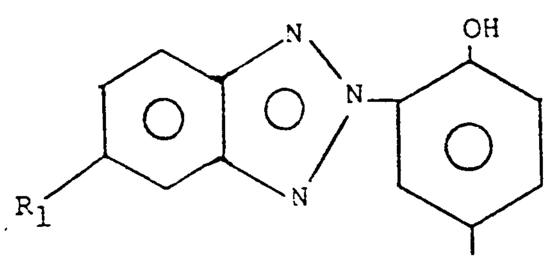

mit einem verzweigten Alken mit 8 bis 30 Kohlenstoffatomen in Gegenwart eines sauren Katalysators und bei einer Temperatur von 100-200 ° C.

$R_1$ steht vorzugsweise für Wasserstoff.

$R_2$ bedeutet bevorzugt Alkyl mit 8 bis 16 Kohlenstoffatomen und besonders bevorzugt Alkyl mit 9 bis 12 Kohlenstoffatomen.

Die 2-(2-Hydroxyphenyl)-2H-benztriazollichtabsorptionsmittel werden in herkömmlicher Weise durch Kuppeln eines entsprechend substituierten Phenols mit einem o-Nitrophenyldiazoniumsalz zu einem o-Nitroazobenzolzwischenprodukt, das nachfolgend reduziert und zum entsprechenden 2H-Benztriazol ringgeschlossen wird, erhalten.

Es ist offensichtlich, dass jede Aenderung in der Art der Substitution am Phenolteil, beispielsweise um die Eigenschaften des fertigen 2H-Benztriazols zu modifizieren, am Phenolmolekül selbst durchgeführt

werden muss, bevor man mit der herkömmlichen 2H-Benztriazolsynthese beginnt. Dies erfordert eine oder mehrere zusätzliche Stufen in der Synthesefolge für jedes neue 2H-Benztriazolprodukt. Ausserdem treten bei diesen Stufen unvermeidliche Nebenreaktionen auf, welche die Einfügung mindestens eines Kristallisationsschritts erforderlich machen, damit man ein Produkt annehmbarer Reinheit erhält.

Die obige Arbeitsweise eignet sich nur schlecht zur Herstellung nicht-kristalliner oder flüssiger Produkte, bei denen eine Reinigung durch Kristallisation unmöglich ist.

Bei dem in U.S. Patent 4 129 521 offenbarten Verfahren wird in der Tat beschrieben, dass es zur Herstellung flüssiger Produkte in annehmbarer Reinheit notwendig ist, (1) das rohe 2H-Benztriazolprodukt im Vakuum zu destillieren, das einmal destillierte Produkt zur Entfernung verschiedener unerwünschter Verunreinigungen mit Essigsäureanhydrid zu behandeln, das acetylierte Gemisch einer zweiten Vakuumdestillation zu unterwerfen, das Destillat stundenlang bei erhöhten Temperaturen mit Luft auszublasen und schliesslich das Material unter Molekulardestillationsbedingungen noch ein drittes Mal zu destillieren. Nur nach diesen umständlichen und wirtschaftlich aufwendigen Schritten erhält man ein flüssiges, als Lichtabsorptionsmittel verwendbares Produkt.

Es bestand daher offensichtlich ein Bedarf nach einer besseren Methode zur Herstellung flüssiger oder nichtkristalliner 2H-Benztriazole, da der herkömmliche Weg der Herstellung eines alkylierten Phenols und danach des Benztriazols daraus eine fast unmögliche Aufgabe bei der Entfernung unerwünschter Verunreinigungen aus dem Benztriazol in praktisch durchführbarer Weise darstellt.

Der Weg über eine Alkylierung eines vorgebildeten 2H-Benztriazols wurde nicht als erfolgversprechend angesehen, da es bekannt war, dass in ortho-Stellung durch eine 2H-Benztriazolylfunktion substituierte Phenole für elektrophile Substitution (= Alkylierung) am Phenolring stark desaktiviert sind.

Es war deshalb überraschend, dass sich eine direkte Alkylierung am Phenolring vorgebildeter 2H-Benztriazole zu den erwünschten gemischten alkylierten Produkten als in glatter und direkter Weise durchführbar erwies.

Die direkte Alkylierung von 2-(2-Hydroxy-5-methylphenyl)-2H-benztriazol mit einem α-Olefin bzw. geradkettigem Alken oder mit einem verzweigten Alken fand somit nicht nur überhaupt statt, sondern erfolgte überdies mit ausgezeichneten Umsätzen (über 90%) des vorgebildeten Benztriazols zu alkylierten Produkten.

Da das 2-(2-Hydroxy-5-methylphenyl)-2H-benztriazol in der para-Stellung zur Hydroxylgruppe bereits substituiert ist, wird eine Alkylierung in ortho-Stellung zur Hydroxylfunktion zu einem Gemisch aus 2-(2-Hydroxy-3-höherverzweigtalkyl-5-methylphenyl)-2H-benztriazolen erzwungen.

Die Art der in die Benztriazole eingeführten höherverzweigten Alkylgruppen ist von dem zur Alkylierung eingesetzten Alkentyp abhängig. Der Einsatz eines α-Olefins bzw. geradkettigen Alkens bewirkt die Einführung verzweigter sekundärer Alkylgruppen, während der Einsatz verzweigter Alkene zu verzweigten Alkylgruppen mit einer Mehrzahl Alkylverzweigungen entlang der Hauptalkylkette führt.

Insbesondere ist das erste Verfahren eine Herstellungsmethode für flüssige oder nicht-kristalline Gemische von Benztriazolen, welche im wesentlichen aus Verbindungen der Formel

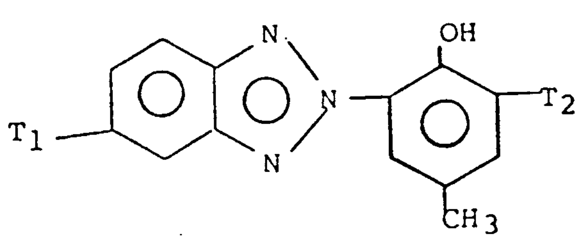

(I)

bestehen, worin $T_1$ Wasserstoff oder Chlor und $T_2$ ein statistisches Gemisch aus mindestens drei isomeren verzweigten sekundären Alkylgruppen mit je 8 bis 30 Kohlenstoffatomen der Formel

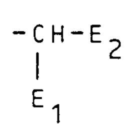

worin $E_1$ für geradkettiges Alkyl mit 1 bis 14 Kohlenstoffatomen und $E_2$ für geradkettiges Alkyl mit 4 bis 15

Kohlenstoffatomen stehen, wobei die Gesamtzahl Kohlenstoffatome in $E_1$ plus $E_2$ 7 bis 29 beträgt, darstellen, welches Verfahren dadurch gekennzeichnet ist, dass man ein Benztriazol der Formel

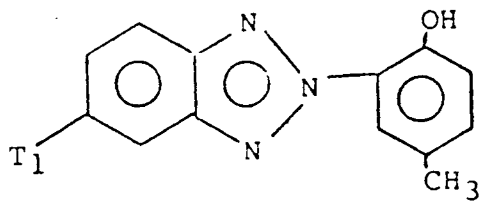

worin $T_1$ Wasserstoff oder Chlor darstellt, in Gegenwart eines sauren Katalysators bei einer Temperatur von 100 bis 200 ° C mit einem geradkettigen Alken mit 8 bis 30 Kohlenstoffatomen alkyliert.

Das zweite Verfahren ist eine Herstellungsmethode für flüssige oder nicht-kristalline Gemische von Benztriazolen, welche im wesentlichen aus Verbindungen der Formel

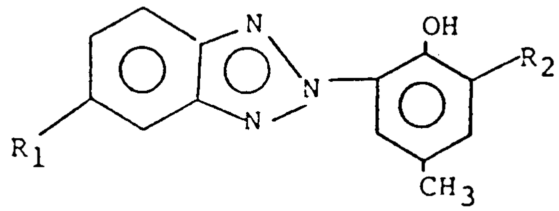

(II)

bestehen, worin $R_1$ Wasserstoff oder Chlor und $R_2$ ein statistisches Gemisch aus mindestens drei isomeren verzweigten Alkylgruppen mit je 8 bis 30 Kohlenstoffatomen und mit einer Mehrzahl Alkylverzweigungen entlang der Hauptalkylkette darstellen, welches Verfahren dadurch gekennzeichnet ist, dass man ein Benztriazol der Formel

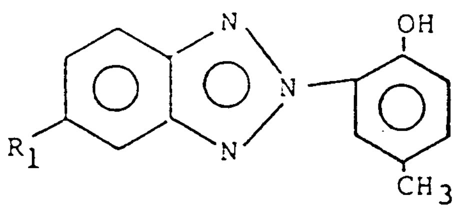

worin $R_1$ Wasserstoff oder Chlor darstellt, in Gegenwart eines sauren Katalysators bei einer Temperatur von 100 bis 200 ° C mit einem verzweigten Alken mit 8 bis 30 Kohlenstoffatomen alkyliert.

Unter diesen scharfen Reaktionsbedingungen unterliegt das Alkylierungsmittel (das geradkettige oder verzweigte Alken) selbst einer chemischen Umformung oder Isomerisierung. Demgemäss stellen die in das Benztriazol eingeführten Alkylsubstituenten nicht eine einzige diskrete funktionelle Gruppe dar, sonder eher ein statistisches Gemisch isomerer Gruppen. Dieses statistische Gemisch von Gruppen ($T_2$ bzw. $R_2$) bedingt eine strukturelle Verschiedenartigkeit, die zu dem flüssigen oder nichtkristallinen physikalischen Zustand der dabei erhaltenen Produkte beiträgt.

Unter den vorliegenden Verfahrensbedingungen wird die Doppelbindung in dem Alkenalkylierungsmittel entlang der Kohlenstoffkette zu einem statistischen Gemisch von Komponenten isomerisiert, die dann an den Phenolring im Benztriazol gebunden werden können.

Mit 1-Octen als dem $\alpha$-Olefin zur Erläuterung besteht das statistische Gemisch von Octylgruppen, die mit $T_2$ als Octyl als $T_2$ eingeführt würden, aus

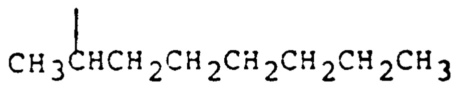

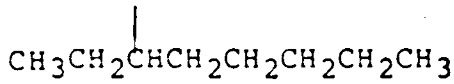

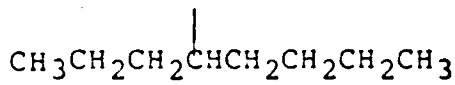

$T_2$ als Octyl würde somit zu mindestens drei in dem hergestellten Benztriazolgemisch vorhandenen Isomeren führen.

Zu den zur Herstellung der vorliegenden Benztriazole benötigten, die $T_2$-Komponente enthaltenden geradkettigen Alkenen gehören $\alpha$-Olefine und geradkettige Alkene mit mittelständiger Doppelbindung. Während der Alkylierungsreaktion wird die Doppelbindung wie oben erläutert entlang der Kohlenstoffkette zu einem statistischen Gemisch verzweigter sekundärer Alkylgruppen der Formel

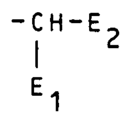

isomerisiert.

Bei diesem Verfahren verwendbare $\alpha$-Olefine sind beispielsweise 1-Octen, 1-Nonen, 1-Decen, 1-Undecen, 1-Dodecen, 1-Tridecen, 1-Tetradecen, 1-Hexadecen, 1-Octadecen, 1-Eikosen, 1-Dokosen, 1-Tetrakosen sowie 1-Triakonten.

Diese $\alpha$-Olefine sind grösstenteils im Handel erhältlich oder werden durch Telomerisierung von Aethylen nach bekannten Methoden hergestellt.

Als geradkettige Alkene mit mittelständiger Doppelbindung kommen beispielsweise 2-Octen, 4-Octen, 5-Decen sowie 9-Trikosen in Betracht.

Diese Alkene sind ebenfalls weitgehend Handelsobjekte.

Zur Herstellung der vorliegenden Benztriazolgemische mit der Komponente $R_2$ werden verzweigte Alkene als Alkylierungsmittel benötigt. Dabei wird ebenfalls die Doppelbindung während der Alkylierungsreaktion entlang der Kohlenstoffkette isomerisiert und wegen der Verzweigung im ursprünglichen Alken können Umlagerungen eintreten. Dabei erhält man ein statistisches Gemisch verzweigter Alkylgruppen mit jeweils einer Anzahl Alkylverzweigungen entlang der Hauptalkylkette.

Bei diesem Verfahren verwendbare verzweigte Alkene sind beispielsweise Dipropylen, Tripropylen, Tetrapropylen, Pentapropylen, Diisobutylen, Triisobutylen, Tetraisobutylen, Pentaisobutylen, 2,2,4,6,6-Pentamethyl-3-hepten, Diisoamylen, Triisoamylen, Tetraisoamylen sowie Pentaisoamylen.

Die meisten dieser hochverzweigten Alkene sind im Handel erhältlich oder lassen sich aus Propylen, Isobutylen oder Isoamylen durch Oligomerisierung mit sauren Katalysatoren herstellen.

Dass dieses Gemisch aus isomeren Resten als $T_2$ oder $R_2$ zur Herstellung eines flüssigen oder nicht-kristallinen Produkts entscheidend ist, ersieht man daraus, dass bei Alkylsubstitution mit einem definierten Isomeren feste kristalline Produkte erhalten werden. Zum Beispiel schmilzt 2-(2-Hydroxy-3,5-di-tert.-octylphenyl)-2H-benztriazol bei 105-106 °C.

Die Alkylierungsverfahren lassen sich über einen Bereich von Bedingungen bezüglich Zeit, Temperatur, Verhältnis von Olefin zu Benztriazol, Katalysatoren und Katalysatorkonzentrationen durchführen.

Man muss die Alkylierung genügend lang ablaufen lassen, üblicherweise ungefähr 4 Stunden lang, doch wird die Ausbeute an alkyliertem Produkt durch Reaktionszeiten über 12 Stunden nicht erhöht. Vorzugsweise erfolgt die Alkylierungsreaktion für einen Zeitraum von 6-8 Stunden.

Verhältnismässig scharfe Reaktionsbedingungen sind erforderlich, da der Phenolring des 2H-Benztriazolausgangsprodukts desaktiviert ist. Die Reaktionstemperaturen können 100 bis 200 °C betragen. Temperaturen unter 140 °C ergeben niedrigere Ausbeuten an alkyliertem Produkt, und Temperaturen über 180 °C führen zu Produkten schlechterer Qualität in niedrigeren Ausbeuten. Vorzugsweise lässt man das Verfahren bei 140 bis 170 °C und besonders bevorzugt bei 160-165 °C ablaufen, wobei man Ausbeuten über 90%

erhält.

Um 2-(2-Hydroxy-5-methylphenyl)-2H-benztriazol zu alkylieren, muss mindestens 1 Aequivalent Alken pro Aequivalent 2H-Benztriazol vorhanden sein. Da unter diesen Reaktionsbedingungen konkurrierende Reaktionen mit dem Alken, wie Dimerisierung, Oligomerisierung oder Polymerisation, ebenfalls möglichsind, erhält man bei Anwendung eines 1:1 Aequivalenzverhältnisses von Alken:Benztriazol üblicherweise eine Ausbeute unter 40% an erwünschtem alkyliertem Produkt.

Durch Erhöhung der Alkenkonzentration gegenüber Benztriazol auf ein 4:1 Aequivalenzverhältnis werden die Ausbeuten an alkylierten Produkten stark erhöht, bis auf über 85%.

Höhere Alkenüberschüsse wie ein 6:1 Aequivalenzverhältnis steigern die Ausbeuten nicht mehr weiter. Vorzugsweise beträgt das Aequivalenzverhältnis Alken:Benztriazol in den vorliegenden Verfahren 3,5 bis 4,5:1.

Der saure Katalysator wird aus der aliphatische, aromatische und substituierte aromatische Sulfonsäuren, Schwefelsäure, Phosphorsäure, saure Tone und heterogene saure Katalysatoren (Molekularsiebe) umfassenden Gruppe ausgewählt.

Die bei dem vorliegenden Verfahren anzuwendende Katalysatorkonzentration beträgt 0,2 bis 3 Aequivalente Katalysator pro Aequivalent Benztriazol, vorzugsweise 0,3 bis 2 Aequivalente und besonders bevorzugt 0,5 bis 1 Aequivalent sauren Katalysator pro Aequivalent Benztriazol.

Als Sulfonsäuren kommen beispielsweise Methansulfonsäure, Aethansulfonsäure, Butansulfonsäure, Benzolsulfonsäure, Toluolsulfonsäure und Dodecylbenzolsulfonsäure in Betracht. Aliphatische Sulfonsäuren sind vorzuziehen.

Handelsübliche saure aktivierte Tone wie Filtrol XJ-8303, Filtrol XJ-8405, Filtrol 22, Filtrol 4 und Filtrol 13 sind ebenfalls wirksame Alkylierungskatalysatoren in den vorliegenden Verfahren.

Methansulfonsäure wird ganz besonders bevorzugt als Katalysator.

Schutz gegen durch UV-Licht verursachte Schädigungen ist von besonderer Bedeutung in der photographischen Technik und vor allem in der Farbphotographietechnik.

Um die in einem farbphotographischen Material vorhandenen Komponenten (insbesondere Farbstoffe und Kuppler) möglichst wirksam gegen Zerstörung durch ultraviolettes Licht zu schützen, werden üblicherweise UV-Absorber in eine oder mehrere der oberen Schichten des Materials eingebracht. In der Regel erfolgt dies dadurch, dass man den UV-Absorber in einem hochsiedenden Lösungsmittel löst und diese Lösung in Form sehr feiner Tröpfchen in der entsprechenden Giesslösung dispergiert. Da diese Tröpfchen die mechanischen Eigenschaften der Schicht beeinträchtigen und "ausschwitzen" können, wenn sie in der Deckschicht des Materials vorliegen, kommt es darauf an, die Menge an Absorberlösung so klein wie möglich zu halten. Dies ermöglicht es auch, dünnere Schichten herzustellen, was wiederum Vorteile bei der Verarbeitung ergibt (Verschleppung zwischen Bädern und Trocknen). Es ist deshalb wünschenswert, solche UV-Absorber einzusetzen, die die höchstmögliche Löslichkeit in den üblichen hochsiedenden Lösungsmitteln aufweisen. Die UV-Absorber des Standes der Technik, z.B. die aus der japanischen Anmeldung Sho 54-95 233 bekannten Stabilisatoren, erfüllen diese Forderung nicht in genügendem Ausmass.

Es wurde nun gefunden, dass die vorliegenden Produkte, da sie flüssig oder nicht-kristallin sind, ohne Verwendung begleitender hochsiedender Lösungsmittel oder nur mit der kleinsten Menge davon in farbphotographischem Material verwendet werden können. Zudem sind diese Verbindungen weitgehend nicht flüchtig und schwitzen nicht aus.

Eine typische photographische Zusammensetzung besteht aus einem Papierträger, auf dem eine oder mehrere lichtempfindliche Schichten gegossen sind und eine den Ultraviolettlichtabsorber in einem Bindemittel enthaltende Schicht so angeordnet ist, dass sie die Schutz erfordernde(n) Schicht oder Schichten schützt.

Es ist wohlbekannt, dass ultraviolette Strahlung eine schädigende Wirkung auf photographische Schichten ausübt. Ultraviolette Strahlung in den für Belichtung photographischer Produkte verwendeten Lichtquellen führt manchmal zu unerwünschter Belichtung der Schicht(en) eines photographischen Elements. Dies gilt insbesondere für zur Verwendung in der Farbphotographie bestimmte photographische Elemente, in denen die Emulsion für längerwellige Bereiche sensibilisiert ist und nur eine Aufnahme der Strahlung im sichtbaren Spektrum erwünscht ist.

Farbphotographien auf mehrschichtigem photographischem Material, insbesondere solche, in denen die Farbstoffbilder durch Farbentwicklung in empfindlichen Emulsionsschichten erzeugt werden, sind gegen Verblassen und Verfärbung unter der Einwirkung ultravioletter Strahlung, der die Photographien beim Betrachten ausgesetzt werden, anfällig. Die in der Emulsionsschicht nach der Erzeugung der Bildaufnahmen enthaltenen restlichen Kuppler unterliegen einem Angriff durch ultraviolette Strahlung, was zu unerwünschtem Schleier in der fertigen Photographie führt. Die Wirkung ultravioletter Strahlung auf fertige Farbphotographien macht sich besonders bei Positivkopien auf Papier oder einem sonstigen undurchsichtigen Träger bemerkbar, da diese Art Kopie häufig im Tageslicht betrachtet wird, das einen hohen Gehalt an ultravioletter Strahlung aufweist. Die verblassenden und verfärbenden Wirkungen auf Farbstoffe werden anscheinend vor allem durch jene Lichtwellenlängen verursacht, die dem sichtbaren Bereich des Spektrums naheliegen, d.h. 300-400 nm.

Es ist bekannt, dass sich photographische Silberhalogenidmaterialien durch Einbau nicht-diffundierender ultraviolettabsorbierender Verbindungen in die Silberhalogenidemulsionsschichten oder darüberliegende Kolloidüberzüge vor ultravioletter Strahlung schützen lassen.

Eine grosse Zahl Ultraviolettabsorber wurden dafür vorgeschlagen. Ultraviolettabsorbierende Verbindungen zur photographischen Anwendung müssen im allgemeinen farblos oder fast farblos sein, gute Verträglichkeit mit dem Einbaumedium besitzen, gegenüber sonstigen photographischen Zusatzstoffen im Element und in der Verarbeitungslösung indifferent sein, gutes Ultraviolettabsorptionsvermögen besitzen und gegen ultraviolette Strahlung stabil sein. Zum Einbau in photographische Elemente in Betracht kommende Verbindungen sind z.B. in U.S. Patent 3 253 921 beschrieben.

Aromatische organische Verbindungen wie Ultraviolettabsorber, farbstoffbildende Kuppler, Antischleiermittel, Filterfarbstoffe und dergleichen müssen zur Entfaltung ihrer Wirkung nicht-diffundierend und in hochdisperser Form genügend fein in den wässrigen photographischen Gelatineschichten verteilt sein.

Dies lässt sich durch verschiedene chemische oder physikalische Methoden einschliesslich der Substitution des organischen Moleküls mit Sulfonsäure- oder sonstigen löslichmachenden Gruppen, durch Tränkung mit einem polaren organischen Lösungsmittel oder durch Lösungsmitteldispergiermethoden erreichen.

Die vorliegenden flüssigen oder nicht-kristallinen 2H Benztriazole sind als Ultraviolettabsorber in photographischen Gelatineschichten ausserordentlich nützlich. Sie besitzen erwünschte Absorptionseigenschaften im ultravioletten Bereich, d.h. maximale Absorption im nahen Ultraviolett und eine scharfe Kante gerade ausserhalb des sichtbaren Bereichs, sind weitgehend farblos, durch entweder Lösungsmitteldispersions- oder Tränkungsmethoden leicht dispergierbar oder auflösbar sowie photographisch indifferent.

Die vorliegenden Verbindungen weisen ausgezeichnete Verträglichkeitseigenschaften in Gelatineschichten der photographischen Zusammensetzung auf, was zu praktisch dunstfreien Zusammensetzungen und dabei hervorragendem Schutz der Farbstoffbilder gegen die schädlichen Einflüsse ultravioletter Strahlung führt. Durch diese Eigenschaftskombination sind die vorliegenden Benztriazollichtabsorptionsmittel eindeutig von der gattungsmässigen Offenbarung in U.S. Patent 3 253 921 abgegrenzt. Diese günstigen Ergebnisse erhält man, wenn die vorliegenden Benztriazole direkt in die Gelatineschicht oder mittels der Lösungsmitteldispergiermethode eingebaut werden.

Eine Aufgabe der Erfindung besteht darin, neue, durch Einbau ultraviolettabsorbierender Materialien gegen die schädlichen Einflüsse ultravioletter Strahlung geschützte photographische Elemente zur Verfügung zu stellen. Es ist eine weitere Aufgabe, in hochstabiler Form eingebaute Ultraviolettabsorber enthaltende farbphotographische Materialien zu schaffen. Noch eine weitere Aufgabe betrifft die Erzeugung eines nicht-diffundierenden Ultraviolettabsorbers.

Gegenstand der Erfindung ist weiterhin ein stabilisiertes organisches Material in Form photographischen Materials oder als Teil eines photographischen Materials, das vorzugsweise in Deckschichten 0,05 bis 5 Gew.-% einer erfindungsgemässen Verbindung, bezogen auf das photographische Material ohne Stabilisator, enthält.

Sind die vorliegenden Verbindungen flüssig, so werden die besagten Benztriazole in ein hydrophiles Kolloid durch Erhitzen einer das flüssige Benztriazol und ein geeignetes Dispergiermittel enthaltenden wässrigen Lösung dieses hydrophilen Kolloids auf eine mässige Temperatur, oberhalb der dieses Benztriazol hoch fliessfähig ist, Rühren des so erhaltenen Gemischs zu einer feinen Dispersion des Benztriazols im Kolloid und nachfolgende Abkühlung des Gemischs eingebaut.

Sind die vorliegenden Verbindungen nicht flüssig bei Raumtemperatur aber nicht-kristallin, so kommt die Anwendung der kleinstmöglichen Menge eines hochsiedenden Lösungsmittels zur Erleichterung der Fliessfähigkeit der Verbindung in Betracht, um die obigen Aufgaben durch die Lösungsmitteldispergiermethode zum Einbau der vorliegenden Verbindungen in wässrige hydrophile Kolloidlösungen zum Giessen von Silberhalogenidemulsionsschichten oder dazugehörigen hydrophilen Kolloidschichten zu lösen.

Zu bevorzugten hochsiedenden Lösungsmitteln zählen Di-n-butylphthalat, Benzylphthalat, Triphenylphosphat, Tri-o-kresylphosphat, Diphenyl-mono-p-tert.-butylphenylphosphat, Monophenyl-di-p-tert.-butylphenylphosphat, Diphenyl-mono-o-chlorphenylphosphat, Monophenyl-di-o-chlorphenylphosphat, Tri-p-tert.-butylphenylphosphat, Tri-o-phenylphenylphosphat, Di-p-tert.-butylphenyl-mono-(5-tert.-butyl-2-phenylphenyl)-phosphat usw.

Zweckmässige hydrophile Kolloide oder Bindemittel sind unter anderem Gelatine, Albumin, usw.,

Cellulosederivate, Polyvinylverbindungen usw. Zu polymeren Bindemitteln gehören Polyvinylalkohol bzw. hydrolysiertes Polyvinylacetat, ein weitgehend hydrolysierter Celluloseester wie auf einen Acetylgehalt von 19-26 Prozent hydrolysiertes Celluloseacetat, wasserlösliches Aethanolamincelluloseacetat, Polyacrylamid mit einem kombinierten Acrylamidgehalt von 30-60 Prozent und einer spezifischen Viskosität von 0,25-1,5 auf einem imidierten Polyacrylamid ähnlichen Acrylamidgehalts und ähnlicher Viskosität, Vinylalkoholpolymere mit Urethancarbonsäuregruppen dieser Art oder mit Cyanacetylgruppen, wie ein Vinylalkohol/Vinylcyanacetatcopolymer, oder ein durch Polymerisation eines Proteins oder gesättigten acylierten Proteins mit einem eine Vinylgruppe tragenden Monomeren erhaltenes polymeres Material.

Die Dispersion einer vorliegenden Verbindung im Bindemittelmaterial wird über die lichtempfindliche Schicht des photographischen Elements gegossen. Ist das photographische Element ein zur Verwendung in der Farbphotographie bestimmtes Material, so braucht die Ultraviolettfilterschicht nicht eine Aussenschicht zu sein, sondern kann als Zwischenschicht eingesetzt werden, d.h. unter der Schicht bzw. den Schichten, die keinen Schutz erfordern, und über der bzw. den Schutz benötigenden Schicht(en). Beispielsweise in einem aus drei verschieden sensibilisierten Schichten bestehenden Mehrschichtenmaterial, wobei die rotempfindliche Schicht direkt am Träger, die grünempfindliche Schicht darüber auf der rotempfindlichen Schicht und die blauempfindliche Schicht gegenüber den übrigen lichtempfindlichen Schichten ganz aussen liegt, kann man die Ultraviolettfilterschicht zwischen den blau- und grünempfindlichen Schichten oder zwischen den grün- und rotempfindlichen Schichten anordnen. Aehnlich kann man in einem anderen photographischen Element, in dem die Schichten umgekehrt sind, d.h. die blauempfindliche Schicht ist auf den Träger gegossen und die grün- und rotempfindlichen Schichten liegen über der blauempfindlichen Schicht in dieser Reihenfolge, die Ultraviolettfilterschicht über allen drei Schichten oder zwischen jeweils zwei von diesen Schichten anordnen. Auch kann man die ultraviolettabsorbierende Zusammensetzung direkt in die lichtempfindliche Emulsion anstatt oder zusätzlich zu einer anderen Schicht einarbeiten. Die Menge an ultraviolettabsorbierendem Material lässt sich variieren, je nach der erwünschten Wirkung und dem Anwendungszweck des Materials.

Die ultraviolettabsorbierenden Zusammensetzungen werden in weiten Konzentrationsbereichen gegossen, üblicherweise im Bereich von 65 bis 1000 mg ultraviolettabsorbierender Verbindung pro $m^2$ photographisches Element. Der bevorzugte Bereich ist 250 bis 525 $mg/m^2$. Die optimalen Giesskonzentrationen sind dabei von dem jeweils zu schützenden photographischen Element und dem gewünschten Schutzumfang abhängig. Die optimalen Giesskonzentrationen für ein gegebenes photographisches Element lassen sich nach dem Stand der Technik wohlbekannten Methoden bestimmen.

Jegliches photographische Element kann vorteilhaft erfindungsgemäss geschützt werden. Der Träger in diesen photographischen Elementen kann aus irgendeinem der herkömmlichen Trägermaterialien bestehen, wie festen Trägern z.B. Celluloseacetat usw., lichtundurchlässigen Trägern wie weisspigmentiertem Film, Papier und dergleichen.

Die vorliegenden ultraviolettabsorbierenden Verbindungen zeichnen sich durch ihre Nichtdiffundierbarkeit in gegossenen Schichten, gute Stabilität in den Einführlösungsmitteln und ihre gute Ultraviolettabsorption aus. Ultraviolettabsorbierende Schichten mit den vorliegenden, nach den bevorzugten erfindungsgemässen Methoden eingebauten Verbindungen besitzen eine unerwartet hohe Stabilität bei längerer Belichtung mit ultravioletter Strahlung, was sie ideal geeignet zum Schutz photographischer Elemente, insbesondere Farbstoffbilder in Farbmaterialien macht.

Die vorliegenden flüssigen Benztriazole lassen sich zweckmässig in photographischen Elementen zusammen mit anderen flüssigen Ultraviolettabsorbern (UVA) verwenden, wie 5-Chlor-2-[2-hydroxy-3-tert.-butyl-5-(2-octyloxyäthyl)-phenyl]-2H-benztriazol.

Die vorliegenden flüssigen Benztriazole sind auch als Lösungsmittel für andere feste UVA-Materialien oder für andere Komponenten in einem Silberhalogenidphotographieelement verwendbar,entweder für sich oder als Kombination mit üblichen photographischen Oelen, wie in den europäischen Patentanmeldungen 84 692 und 84 694 beschrieben.

Zu solchen anderen Verbindungen gehören

- Gelb-, Purpur- und Blaugrünkuppler,
- DIR-Kuppler, Schwarzkuppler, farblose Kuppler,
- Stabilisatoren für chromogene Kuppler,
- Stabilisatoren für chromogene Farbstoffe,
- Schirmfarbstoffe, Antihalofarbstoffe, Farbbleichfarbstoffe,
- Formaldehydabfangmittel,
- Sensibilisierfarbstoffe,
- optische Aufheller,
- Abfangmittel für oxydierten Entwickler,

- Verbindungen, die bei der Entwicklung diffundierbare Farbstoffe freisetzen, und
- Elektronenübertragungsmittel.

Beispiele für weitere, kombiniert mit den vorliegenden Verbindungen in Frage kommende UVA-Materialien sind unter anderen

1. Benzophenone
2,4-Dihydroxy-benzophenon 2-Hydroxy-4-äthoxy-benzophenon
2,2'-Dihydroxy-4-methoxy-benzophenon
2-Hydroxy-4-n-octoxy-benzophenon
2-Hydroxy-4-isooctoxy-benzophenon
2-Hydroxy-4-dodecyloxy-benzophenon
2. Benztriazole:
2-(2-Hydroxy-5-methylphenyl)-benztriazol,
2-(2-Hydroxy-3,5-di-t-butylphenyl)-benztriazol,
2-(2-Hydroxy-3-t-butyl-5-äthylphenyl)-5-chlorbenztriazol,
2-(2-Hydroxy-3,5-di-t-butylphenyl)-5-chlorbenztriazol,
2-(2-Hydroxy-3,5-di-t-amylphenyl)-benztriazol,
2-(2-Hydroxy-3-s-butyl-5-t-butylphenyl)-benztriazol,
2-(2-Hydroxy-5-t-butylphenyl)-benztriazol,
2-(2-Hydroxy-5-t-octylphenyl)-benztriazol,
Gemisch aus 50% 2-[(2-Hydroxy-3-t-butyl-5-(2"-n-octoxycarbonyl)-äthyl)-phenyl]-5-chlorbenztriazol und 50% 2-[(2-Hydroxy-3-t-butyl-5-(2"-äthylhexyloxy)-carbonyl)-äthyl)-phenyl]-5-chlorbenztriazol,
2-[2-Hydroxy-3,5-di-($\alpha$,$\alpha$-dimethylbenzyl)-phenyl]-benztriazol
3. Benzylidenmalonate
Methyl-2-carboxymethyl-3-(4'-methoxyphenyl)-acrylat
4. Salicylate
p-Octylphenylsalicylat
Phenylsalicylat
t-Butylphenylsalicylat
5. Monobenzoate
Resorcin-monobenzoat
3,5-Di-t-butyl-4-hydroxybenzoesäure-hexadecylester
6. Oxamide
5-t-Butyl-2-äthoxy-2'-äthyloxanilid,
2-Aethoxy-2'-äthyloxanilid
7. 5-Dialkylamino-2,4-pentadiensäurester
5-Diäthylamino-2-phenylsulfonyl-2,4-pentadiensäurehendecylester
8. 5-Dialkylamino-2-cyan-2,4-pentadien-nitrile
5-Dihexylamino-2-cyan-2,4-pentadien-nitril
9. 2,4-Di-t-butylphenyl-3,5-di-t-butyl-4-hydroxybenzoesäure ester,
3,5-Di-t-butyl-p-hydroxybenzoesäure,
Di-(1,2,2,6,6-pentamethyl-4-piperidinyl)-butyl-(3',5'-di-t-butyl-4-hydroxybenzyl)-malonat,
Bis-(1,2,6,6-tetramethyl-4-piperidinyl)-sebacinat,
Bis-(1,2,2,6,6-pentamethyl-4-piperidyl)-sebacinat,
Butantetracarbonsäure-tetra-(2,2,6,6-tetramethyl-4-piperidinyl)-ester.

Die vorliegenden Verbindungen sind auch in Blaugrünschichten zusammen mit entweder Phenol-, Naphthol- oder 2,5-Diacylaminophenolkupplern oder mit Gemischen dieser Kuppler zwecks Verhütung von Bildverblassung und -verfärbung verwendbar.

Die Anwendung bekannter Benztriazole in solchen Systemen ist aus den japanischen Offenlegungsschriften Sho 58-221 844 und 59-46 646 bekannt.

Die erfindungsgemässen Verbindungen sind wirksame Lichtschutzmittel in zahlreichen organischen Polymeren. Zu stabilisierende Polymere sind unter anderen:

1. Sich von Mono- oder Diolefinen ableitende Polymere, z.B. gegebenenfalls vernetztes Polyäthylen sowie Polypropylen, Polyisobutylen, Polymethylbuten-1, Polymethylpenten-1, Polyisopren und Polybutadien.
2. Gemische der unter 1. angeführten Homopolymeren, zum Beispiel Gemische aus Polypropylen und Polyäthylen, Polypropylen und Polybuten-1 sowie Polypropylen und Polyisobutylen.
3. Copolymere auf Grundlage der unter 1. angeführten Monomeren, beispielsweise Aethylen/Propylencopolymere, Propylen/Buten-1-copolymere, Propylen/Isobutylencopolymere,

Aethylen/Buten-1-copolymere sowie Terpolymere des Aethylens und Propylens mit einem Dien, z.B. Hexadien, Dicyclopentadien oder Aethylidennorbornen, und Copolymere von $\alpha$-Olefinen, z.B. Aethylen mit Acryl- oder Methacrylsäure, sowie Gemenge solcher Copolymeren mit in Abschnitt 1 und 2 oben beschriebenen Homopolymeren.

4. Polystyrol.

5. Copolymere des Styrols und $\alpha$-Methylstyrols, zum Beispiel Styrol/Butadiencopolymere, Styrol/Acrylnitrilcopolymere, Styrol/Acrylnitril/Methacrylatcopolymere, mit Acrylesterpolymeren schlagzäh gemachte Styrol/Acrylnitrilcopolymere sowie Blockcopolymere, z.B. Styrol/Butadien/Styrol, Styrol/Isopren/Styrol und Styrol/Aethylen-propylen/Styrolblockcopolymere.

6. Pfropfcopolymere des Styrols, beispielsweise ein Pfropfpolymer des Styrols auf Polybutadien, ein Pfropfpolymer des Styrols mit Acrylnitril auf Polybutadien sowie deren Gemische mit den unter 5. angeführten Copolymeren, allgemein als Acrylnitril/Butadien/Styrol- oder ABS-Kunststoffe bezeichnet.

7. Halogenhaltige Vinylpolymere, zum Beispiel Polyvinylchlorid, Polyvinylidenchlorid, Polyvinylfluorid, Polychloropren, chlorierte Kautschuke, Vinylchlorid/Vinylidenchloridcopolymere, Vinylchlorid/Vinylacetatcopolymere und Vinylidenchlorid/Vinylacetatcopolymere.

8. Sich von $\alpha,\beta$-ungesättigten Säuren und deren Derivaten ableitende lineare und vernetzte Polymere wie Polyacrylate und Polymethacrylate, Polyacrylamide und Polyacrylnitril.

9. Sich von ungesättigten Alkoholen und Aminen und deren Acylderivaten bzw. Acetalen ableitende Polymere, wie zum Beispiel Polyvinylalkohol, Polyvinylacetat, Polyvinylstearat, Polyvinylbenzoat, Polyvinylmaleinat, Polyvinylbutyral, Polyallylphthalat, Polyallylmelamin und deren Copolymere mit anderen Vinylverbindungen, z.B. Aethylen/ Vinylacetatcopolymere.

10. Sich von Epoxiden ableitende Homopolymere und Copolymere, zum Beispiel Polyäthylenoxyd oder sich von Bis-glycidyläthern ableitende Polymere.

11. Polyacetale, beispielsweise Polyoxymethylen sowie Aethylenoxyd als Comonomer enthaltende Polyoxymethylene.

12. Polyalkylenoxyde, z.B. Polyoxyäthylen, Polypropylenoxyd oder Polybutylenoxyd.

13. Polyphenylenoxyde und deren Gemenge mit schlagzähem Polystyrol.

14. Polyurethane und Polyharnstoffe wie in Urethanüberzügen.

15. Polycarbonate.

16. Polysulfone.

17. Sich von Diaminen und Dicarbonsäuren und/oder Aminocarbonsäuren bzw. den entsprechenden Lactamen ableitende Polyamide und Copolyamide, wie zum Beispiel Polyamid-6, Polyamid-6/6, Polyamid-6/10, Polyamid-11, Polyamid-12 und Poly-m-phenylen-isophthalamid.

18. Sich von Dicarbonsäuren und Dialkoholen und/oder Hydroxycarbonsäuren bzw. den entsprechenden Lactonen ableitende Polyester, z B. Polyäthylenglykolterephthalat und Poly-1,4-dimethylol-cyclohexanterephthalat.

19. Sich einerseits von Aldehyden und andererseits von Phenolen, Harnstoffen und Melamin ableitende vernetzte Polymere, wie z.B. Phenol/Formaldehyd-, Harnstoff/Formaldehyd- und Melamin/Formaldehydharz.

20. Alkydharze, beispielsweise Glycerin/Phthalsäureharze und deren Gemische mit Melamin/Formaldehydharzen.

21. Sich von Copolyestern gesättigter und ungesättigter Dicarbonsäuren mit mehrwertigen Alkoholen sowie Vinylverbindungen als Vernetzungsmittel ableitende ungesättigte Polyesterharze und auch deren halogenhaltige flammwidrige Modifikationen.

22. Natürliche Polymere, zum Beispiel Cellulose, Kautschuk sowie deren chemisch abgeänderte homologe Derivate, z.B. Celluloseacetate, Cellulosepropionate und Cellulosebutyrate und Celluloseäther wie zum Beispiel Methylcellulose.

Die Stabilisierung von Polyolefinen, Styrolpolymeren, Polyacrylaten, Polyamiden, Polyurethanen, halogenhaltigen Vinylpolymeren, Alkydharzen, Acrylharzduroplasten und Epoxyharzen ist von besonderer Bedeutung, und die vorliegenden Benztriazolgemische sind für diesen Zweck hervorragend geeignet. Beispiele für solche Polymere sind Polyäthylen hoher und niedriger Dichte, Polypropylen, Aethylen/Propylencopolymere, Polystyrol, Styrolblockcopolymere, halogenhaltige Vinylpolymere, lineare (= thermoplastische) und vernetzte (= duroplastische) Polyacrylate und Polyurethane, Alkydharze und Epoxyharze in Form von Ueberzügen, Lacken, Fäden, Filmen, Folien, Klebstoffen, Elastomeren, Schäumen und Formkörpern.

Die vorliegenden Stabilisatoren werden den Substraten in einer Konzentration von 0,05 bis 10 Gew.-%, berechnet auf das zu stabilisierende Material, zugesetzt. Vorzugsweise setzt man dabei 0,1 bis 5 Gew.-% zu.

Der Einbau kann nach der Polymerisation erfolgen, beispielsweise durch Einmischen der Verbindungen und gewünschtenfalls weiterer Zusatzstoffe in die Schmelze nach dem Stand der Technik geläufigen Methoden vor oder während der Formgebung oder durch Aufbringen der gelösten oder dispergierten Verbindungen auf das Polymere, gegebenenfalls mit nachfolgendem Abdampfen des Lösungsmittels.

Die Stabilisatoren können den zu stabilisierenden Substraten auch in Form einer konzentrierten Vormischung, die diese Verbindungen z.B. in einer Konzentration von 2,5 bis 25 Gew.-% enthält, zugesetzt werden.

Die erfindungsgemässen Verbindungen sind zwar dazu verwendbar eine Lichtschutzwirkung zu verleihen, doch werden sie bei der Herstellung stabilisierter Zusammensetzungen häufig mit anderen Stabilisatoren, sogar weiteren Lichtschutzmitteln kombiniert.Die Stabilisatoren können zusammen mit Phenolantioxidantien, Pigmenten, Färbemitteln oder Farbstoffen, Lichtschutzmitteln wie sterisch gehinderten Aminen, Metalldesaktivatoren usw. verwendet werden.

Im allgemeinen werden die erfindungsgemässen Stabilisatoren in Mengen von etwa 0,05 bis etwa 10 Gew.-% der stabilisierten Zusammensetzung eingesetzt, doch schwankt dies in Abhängigkeit von dem jeweiligen Substrat und dem Anwendungszweck. Ein vorteilhafter Bereich erstreckt sich von etwa 0,1 bis etwa 5%.

Die Stabilisatoren der Formel I oder II lassen sich leicht nach herkömmlichen Methoden in jeder zweckmässigen Stufe vor der Herstellung von Formkörpern daraus in die organischen Substrate einarbeiten. Zum Beispiel kann man den Stabilisator mit dem trockenen Polymer mischen oder eine Suspension, Lösung oder Emulsion des Stabilisators mit einer Lösung, Suspension oder Emulsion des Polymeren vermischen. Die erfindungsgemässen stabilisierten Polymerzusammensetzungen können gegebenenfalls auch etwa 0,05 bis etwa 10 Gew.-%, vorzugsweise etwa 0,1 bis etwa 5 Gew.-%, verschiedener herkömmlicher Zusatzstoffe wie den folgenden enthalten, insbesondere Phenolantioxydantien oder Lichtschutzmittel sowie deren Gemische:

1. Antioxydantien

1.1 Einfache 2,6-Dialkylphenole wie zum Beispiel 2,6-Di-tert.-butyl-4-methylphenol, 2-tert.-Butyl-4,6-dimethylphenol, 2,6-Di-tert.-butyl-4-methoxymethylphenol und 2,6-Dioctadecyl-4-methylphenol.

1.2 Derivate alkylierter Hydrochinone wie zum Beispiel 2,5-Di-tert.-butyl-hydrochinon, 2,5-Di-tert.-amylhydrochinon, 2,6-Di-tert.-butyl-hydrochinon, 2,5-Di-tert.-butyl-4-hydroxy-anisol, 3,5-Di-tert.-butyl-4-hydroxy-anisol, 3,5-Di-tert -butyl-4-hydroxyphenyl-stearate und Bis-(3,5-di-tert.-butyl-4-hydroxyphenyl)-adipat.

1.3 Hydroxylierte Thiodiphenyläther wie zum Beispiel 2,2'-Thio-bis-(6-tert.-butyl-4-methylphenol), 2,2'-Thio-bis-(4-octylphenol), 4,4'-Thio-bis-(tert.-butyl-3-methylphenol), 4,4'-Thio-bis-(3,6-di-sek.-amylphenol), 4,4'-Thio-bis-(6-tert.-butyl-2-methylphenol) und 4,4'-Bis-(2,6-dimethyl-4-hydroxyphenyl)-disulfid.

1.4 Alkyliden-bisphenole wie zum Beispiel 2,2'-Methylen-bis-(6-tert.-butyl-4-methylphenol), 2,2'-Methylen-bis-(6-tert.-butyl-4-äthylphenol), 4,4'-Methylen-bis-(6-tert.-butyl-2-methylphenol), 4,4'-Methylen-bis-(2,6-di-tert.-butylphenol), 2,6-Di-(3-tert.-butyl-5-methyl-2-hydroxybenzyl)-4-methylphenol, 2,2'-Methylen-bis-[4-methyl-6-(2-methylcyclohexyl)-phenol], 1,1-Bis-(3,5-dimethyl-2-hydroxyphenyl)-butan, 1,1-Bis-(5-tert.-butyl-4-hydroxy-2-methylphenyl)-butan, 2,2-Bis-(3,5-di-tert.-butyl-4-hydroxyphenyl)-propan, 1,1,3-Tris-(5-tert.-butyl-4-hydroxy-2-methylphenyl)-butan, 2,2-Bis-(5-tert.-butyl-4-hydroxy-2-methylphenyl)-4-n-dodecylmercapto-butan, 1,1,5,5-Tetra-(5-tert.-butyl-4-hydroxy-2-methylphenyl)-pentan und Aethylenglykol-bis-[3,3-bis-(3-tert.-butyl-4-hydroxyphenyl)-butyrat].

1.5 O-, N- und S-Benzylverbindungen wie zum Beispiel 3,5,3',5'-Tetra-tert.-butyl-4,4'-dihydroxydibenzyläther, Octadecyl-4-hydroxy-3,5-dimethylbenzyl-mercaptoacetat, Tris-(3,5-di-tert.-butyl-4-hydroxybenzyl)-amin und Bis-(4-tert.-butyl-3-hydroxy-2,6-dimethylbenzyl)-dithioterephthalat.

1.6 Hydroxybenzylierte Malonate wie zum Beispiel Dioctadecyl-2,2-bis-(3,5-di-tert.-butyl-2-hydroxybenzyl)-malonat, Dioctadecyl-2-(3-tert.-butyl-4-hydroxy-5-methylbenzyl)-malonat, Di-dodecylmercaptoäthyl-2,2-bis-(3,5-di-tert.-butyl-4-hydroxybenzyl)-malonat und Di-[4-(1,1,3,3-tetramethylbutyl)-phenyl]-2,2-bis-(3,5-di-tert.-butyl-4-hydroxybenzyl)-malonat.

1.7 Hydroxybenzylaromatische Verbindungen wie zum Beispiel 1,3,5-Tri-(3,5-di-tert.-butyl-4-hydroxybenzyl)-2,4,6-trimethylbenzol, 1,4-Di-(3,5-ditert.-butyl-4-hydroxybenzyl)-2,3,5,6-tetramethylbenzol und 2,4,6-Tri-(3,5-di-tert.-butyl-4-hydroxybenzyl)-phenol.

1.8 s-Triazinverbindungen wie zum Beispiel 2,4-Bis-octylmercapto-6-(3,5-di-tert.-butyl-4-hydroxyanilino)-s-triazin, 2-Octylmercapto-4,6-bis-(3,5-di-tert.-butyl-4-hydroxyanilino)-s-triazin, 2-Octylmercapto-4,6-bis-(3,5-di-tert.-butyl-4-hydroxyphenoxy)-s-triazin, 2,4,6-Tris-(3,5-di-tert.-butyl-4-hydroxyphenoxy)-s-triazin, 2,4,6-Tris-(3,5-di-tert.-butyl-4-hydroxyphenyläthyl)-s-triazin und 1,3,5-Tris-(3,5-di-tert.-butyl-4-hydroxybenzyl)-isocyanurat.

1.9 Amide von β-(3,5-Di-tert.-butyl-4-hydroxyphenyl)-propionsäuren wie zum Beispiel 1,3,5-Tris-(3,5-di-tert.-butyl-4-hydroxyphenyl-propionyl)-hexahydro-s-triazin, N,N'-Di-(3,5-di-tert.-butyl-4-hydroxyphenyl-propionyl)-hexamethylendiamin und N,N'-Bis-β-(3,5-di-t-butyl-4-hydroxyphenyl)-propionylhydrazin.

1.10 Ester der β-(3,5-Di-tert.-butyl-4-hydroxyphenyl)-propionsäure mit einwertigen oder mehrwertigen Alkoholen wie zum Beispiel mit Methanol, Aethanol, Octadecanol, 1,6-Hexandiol, 1,9-Nonandiol, Aethylenglykol, 1,2-Propandiol, Diäthylenglykol, Thiodiäthylenglykol, Neopentylglykol, Pentaerythrit, 3-Thiaundecanol, 3-Thiapentadecanol, Trimethylhexandiol, Trimethyloläthan, Trimethylolpropan, Tris-Hydroxyäthylisocyanurat und 4-Hydroxymethyl-1-phospha-2,6,7-trioxabicyclo[2.2.2]octan.

1.11 Ester der β-(5-tert.-Butyl-4-hydroxy-3-methylphenyl)-propionsäure mit einwertigen oder mehrwertigen Alkoholen wie zum Beispiel mit Methanol, Aethanol, Octadecanol, 1,6-Hexandiol, 1,9-Nonandiol, Aethylenglykol, 1,2-Propandiol, Diäthylenglykol, Thiodiäthylenglykol, Neopentylglykol, Pentaerythrit, 3-Thiaundecanol, 3-Thiapentadecanol, Trimethylhexandiol, Trimethyloläthan, Trimethylolpropan, Tris-hydroxyäthylisocyanurat und 4-Hydroxymethyl-1-phospha-2,6,7-trioxabicyclo[2.2.2]octan.

1.12 Ester der 3,5-Di-tert.-butyl-4-hydroxyphenylessigsäure mit einwertigen oder mehrwertigen Alkoholen wie zum Beispiel Methanol, Aethanol, Octadecanol, 1,6-Hexandiol, 1,9-Nonandiol, Aethylenglykol, 1,2-Propandiol, Diäthylenglykol, Thiodiäthylenglykol, Neopentylglykol, Pentaerythrit, 3-Thiaundecanol, 3-Thiapentadecanol, Trimethylhexandiol, Trimethyloläthan, Trimethylolpropan, Tris-hydroxyäthylisocyanurat und 4-Hydroxymethyl-1-phospha-2,6,7-trioxabicyclo[2.2.2]octan, insbesondere der Tetrakisester des Pentaerythrits.

1.13 Benzylphosphonate wie zum Beispiel Dimethyl-3,5-di-tert.-butyl-4-hydroxybenzylphosphonat, Diäthyl-3,5-di-tert.-butyl-4-hydroxybenzylphosphonat, Di-octadecyl-3,5-di-tert.-butyl-4-hydroxybenzylphosphonat und Dioctadecyl-5-tert.-butyl-4-hydroxy-3-methylbenzylphosphonat.

2. Lichtschutzmittel

2.1 Ester gegebenenfalls substituierter Benzoesäuren z.B. 3,5-Di-tert.-butyl-4-hydroxybenzoesäure-2,4-di-tert.-butylphenylester oder -octadecylester oder -2-methyl-4,6-di-tert.-butylphenylester.

2.2 Sterisch gehinderte Amine z.B. 4-Benzoyl-2,2,6,6-tetramethylpiperidin, 4-Stearyloxy-2,2,6,6-tetramethylpiperidin, Bis-(2,2,6,6-tetramethylpiperidyl)-sebacinat, Bis-(1,2,2,6,6-pentamethylpiperidyl)-sebacinat, Bis-(1,2,2,6,6-pentamethylpiperidyl)-2-n-butyl-2-(2-hydroxy-3,5-di-tert.-butylbenzyl)-malonat oder 3-n-Octyl-7,7,9,9-tetramethyl-1,3,8-triazaspiro[4.5]decan-2,4-dion.

2.3 Oxalsäurediamide z.B. 4,4'-Di-octyloxy-oxanilid, 2,2'-Di-octyloxy-5,5'-di-tert.-butyl-oxanilid, 2,2'-Di-dodecyloxy-5,5'-di-tert.-butyl-oxanilid, 2-Aethoxy-2'-äthyl-oxanilid, N,N'-Bis-(3-dimethyl-aminopropyl)-oxalamid,2-Aethoxy-5-tert.-butyl-2'-äthyl-oxanilid und dessen Gemisch mit 2-Aethoxy-2'-äthyl-5,4'-di-tert.-butyl-oxanilid oder Gemische von o- und p-Methoxy- sowie o- und p-Aethoxydisubstituierten Oxaniliden.

3. Metalldesaktivatoren z.B. Oxanilid, Isophthalsäuredihydrazid, Sebacinsäure-bis-phenylhydrazid, Bis-benzylidenoxalsäuredihydrazid, N,-N'-Diacetyl-adipinsäuredihydrazid, N,N'-Bis-salicyloyloxalsäuredihydrazid, N,N'-Bis-salicyloylhydrazin, N,N'-Bis-(3,5-di-tert.-butyl-4-hydroxyphenylpropionyl)-hydrazin, N-Salicyloyl-N'-salicylal-hydrazin, 3-Salicyloyl-amino-1,2,4-triazol oder N,N'-Bis-salicyloyl-thiopropionsäuredihydrazid.

4. Basische Costabilisatoren z.B. Alkali- und Erdalkalisalze höherer Fettsäuren, beispielsweise Ca-stearat, Zn-stearat, Mg-behenat, Na-ricinoleat oder K-palmitat.

5. Keimbildungsmittel z.B. 4-tert.-Butylbenzoesäure, Adipinsäure oder Diphenylessigsäure.

6. Phosphite und Phosphonite wie zum Beispiel Triphenylphosphit, Diphenylalkylphosphite, Phenyldialkylphosphite, Tri-(nonylphenyl)-phosphit, Trilaurylphosphit, Tri-octadecylphosphit, 3,9-Isodecyloxy-2,4,8,10-tetraoxa-3,9-diphospha[5.5]undecan und Tetra-(2,4-di-tert.-butylphenyl)-diphenylen-4,4'-bis-(phosphonit).

Weitere in die stabilisierten Zusammensetzungen einarbeitbare Zusatzstoffe sind Thiosynergisten wie Dilauryl-thiodipropionat, Schmiermittel wie Stearylalkohol, Füllstoffe, Asbest, Kaolin, Talkum, Glasfasern, Pigmente, optische Aufheller, Flammschutzmittel sowie Antistatika.

Die nachfolgenden Beispiele dienen nur zur Erläuterung und sind nicht als die Art oder den Umfang der vorliegenden Erfindung in irgendeiner Weise einschränkend auszulegen.

Beispiel 1

2-(2-Hydroxy-3-octyl-5-methylphenyl)-2H-benztriazol

In einem mit Stickstoffeinlass, Rührer, Rückflusskühler und Tropftrichter ausgerüsteten Kolben erhitzt man 225 g 2-(2-Hydroxy-5-methylphenyl)-2H-benztriazol, 65 ml Methansulfonsäure und 156 ml 1-Octen sechs Stunden lang auf 120 ° C. Im Verlauf dieser Zeit setzt man dem Reaktionsgemisch weitere 467 ml 1-Octen zu. Dann wird abgekühlt und zweimal mit je 200 ml methanolischer Kalilauge (40 g Kaliumhydroxyd in 100 ml Methanol) extrahiert. Die Extrakte werden mit 1 x 400 ml und dann mit 4 x 200 ml Heptan gewaschen. Man versetzt die Heptanextrakte mit Wasser (200 ml) und trennt die Heptanoberschicht ab, wäscht sie mit 5 x 200 ml wässrigem Methanol (2 Methanol:1 Wasser). Die gewaschene Heptanschicht wird über wasserfreiem Magnesiumsulfat getrocknet und mit 1 x 10 g und dann 1 x 5 g saurem absorbierenden Ton (FILTROL 13) behandelt. Nach Entfernung des Tonabsorptionsmittels wird die Heptanlösung im Vakuum bei 110 ° C/2 mm abgestreift, was 23 g des oben genannten Produkts als gelbe Flüssigkeit liefert.
Analyse:
Berechnet für $C_{21}H_{27}N_3O$: C, 74,7; H, 8,0; N, 12,4.
Gefunden: C, 74,3; H, 7,9; N, 11,8.
Formelmasse: 337,4.
Durch Titration gefundene Masse: 337.

Auf die gleiche Weise wird 5-Chlor-2-(2-hydroxy-3-octyl-5-methylphenyl)-2H-benztriazol hergestellt, jedoch unter Ersatz des oben verwendeten Benztriazols durch eine äquivalente Menge 5-Chlor-2-(2-hydroxy-5-methylphenyl)-2H-benztriazol.

Beispiel 2

2-(2-Hydroxy-3-decyl-5-methylphenyl)-2H-benztriazol

Gemäss der allgemeinen Arbeitsweise nach Beispiel 1 erhitzt man 225 g 2-(2-Hydroxy-5-methylphenyl)-2H-benztriazol, 65 ml Methansulfonsäure und 188 ml 1-Decen 5 Stunden lang auf 160 ° C, währenddessen man allmählich weitere 564 ml 1-Decen dazugibt. Das Gemisch wird dann abgekühlt und das Produkt nach der in Beispiel 1 beschriebenen Methode isoliert, was 183 g des oben angegebenen Produkts als gelbe Flüssigkeit liefert.
Analyse:
Berechnet für $C_{23}H_{31}N_3O$: C, 75,6; H, 8,55; N, 11,5.
Gefunden: C, 75,6; H, 8,7; N, 11,5.
Formelmasse: 365.
Durch Titration gefundene Masse: 394.

Auf die gleiche Weise wird 5-Chlor-2-(2-hydroxy-3-decyl-5-methylphenyl)-2H-benztriazol hergestellt, jedoch unter Ersatz des oben verwendeten Benztriazols durch eine äquivalente Menge 5-Chlor-2-(2-hydroxy-5-methylphenyl)-2H-benztriazol.

Beispiel 3

2-(2-Hydroxy-3-dodecyl-5-methylphenyl)-2H-benztriazol

Gemäss der allgemeinen Arbeitsweise nach Beispiel 1 erhitzt man 225 g 2-(2-Hydroxy-5-methylphenyl)-2H-benztriazol, 65 ml Methansulfonsäure und 222 ml 1-Dodecen vier Stunden lang auf 160 ° C, währenddessen man weitere 666 ml 1-Dodecen dem Reaktionsgemisch zusetzt. Dann kühlt man ab und isoliert das Produkt nach der im Beispiel 1 beschriebenen Methode, was 187 g des oben genannten Produkts als gelbes Oel ergibt.
Analyse:
Berechnet für $C_{25}H_{35}N_3O$: C, 76,3; H, 9,0; N, 10,7.
Gefunden: C, 76,4; H, 9,0; N, 10,5.
Formelmasse: 393,5.
Durch Titration gefundene Masse: 395.

Auf die gleiche Weise wird 5-Chlor-2-(2-hydroxy-3-dodecyl-5-methylphenyl)-2H-benztriazol hergestellt, jedoch unter Ersatz des oben verwendeten Benztriazols durch eine äquivalente Menge 5-Chlor-2-(2-hydroxy-5-methylphenyl)-2H-benztriazol.

Beispiel 4

2-(2-Hydroxy-3-hexadecyl-5-methylphenyl)-2H-benztriazol

Gemäss der allgemeinen Arbeitsweise nach Beispiel 1 erhitzt man 225 g 2-(2-Hydroxy-5-methylphenyl)-2H-benztriazol, 65 ml Methansulfonsäure und 222 ml 1-Hexadecen sechs Stunden lang auf 160 ° C, währenddessen man weitere 865 ml 1-Hexadecen dem Reaktionsgemisch zusetzt. Dann kühlt man ab und isoliert das Produkt nach der im Beispiel 1 beschriebenen allgemeinen Methode. Die Heptanlösung des Produkts wird bei 280 ° C/2 mm zur Entfernung des Heptans und gegebenenfalls zurückgebliebenen 1-Hexadecens abgestreift. Dann löst man den Rückstand in Petroläther zwecks Behandlung mit dem sauren absorbierenden Ton. Danach wird der Petroläther abgestreift, was 124,5 g des oben genannten Produkts als orangefarbene Flüssigkeit ergibt.

Analyse:
Berechnet für $C_{29}H_{43}N_3O$: C, 77,5; H, 9,6; N, 9,3.
Gefunden: C, 77,2; H, 9,3; N, 9,0.
Formelmasse: 449.
Durch Titration gefundene Masse: 483.

Auf die gleiche Weise wird 5-Chlor-2-(2-hydroxy-3-hexadecyl-5-methylphenyl)-2H-benztriazol hergestellt, jedoch unter Ersatz des oben verwendeten Benztriazols durch eine äquivalente Menge 5-Chlor-2-(2-hydroxy-5-methylphenyl)-2H-benztriazol.

Beispiel 5

2-(2-Hydroxy-3-eikosyl-5-methylphenyl)-2H-benztriazol und 2-(2-Hydroxy-3-dokosyl-5-methylphenyl)-2H-benztriazol

Gemäss der allgemeinen Arbeitsweise nach Beispiel 1 erhitzt man 225 g 2-(2-Hydroxy-5-methylphenyl)-2H-benztriazol, 65 ml Methansulfonsäure und 368 ml GULFTENE 20-24 (im wesentlichen ein 50/50-Gemisch aus 1-Eikosen und 1-Dokosen) fünf Stunden lang auf 160 ° C, währenddessen man dem Reaktionsgemisch weitere 854 ml GULFTENE 20-24 zusetzt. Dann kühlt man auf etwa 50 ° C ab und versetzt zur Erleichterung der Isolierung des Produkts nach der im Beispiel 1 beschriebenen allgemeinen Methode mit 400 ml Petroläther. Man erhält die beiden oben genannten Produkte in einem 50/50-Gemisch als gelbe Flüssigkeit.

Analyse:
Berechnet für das Gemisch
$C_{34}H_{53}N_3O$; C, 78,6; H, 10,3; N, 8,1.
Gefunden: C, 78,8; H, 10,5; N, 8,0.

Auf die gleiche Weise wird ein Gemisch aus 5-Chlor-2-(2-hydroxy-3-eikosyl-5-methylphenyl)-2H-benztriazol und 5-Chlor-2-(2-hydroxy-3-dokosyl-5-methylphenyl)-2H-benztriazol hergestellt, jedoch unter Ersatz des oben verwendeten Benztriazols durch eine äquivalente Menge 5-Chlor-2-(2-hydroxy-5-methylphenyl)-2H-benztriazol.

Beispiel 6

2-(2-Hydroxy-3-dodecyl-5-methylphenyl)-2H-benztriazol

Gemäss der allgemeinen Arbeitsweise nach Beispiel 1 erhitzt man 225 g 2-(2-Hydroxy-5-methylphenyl)-2H-benztriazol, 65 ml Methansulfonsäure und 225 ml Propylentetramer (4,6,8-Trimethyl-2-nonen und dessen Isomere) sechs Stunden lang auf 160 ° C, währenddessen man dem Reaktionsgemisch weitere 675 ml Propylentetramer zusetzt. Man kühlt ab und isoliert das Produkt nach der in Beispiel 1 beschriebenen allgemeinen Methode, was 30,9 g des oben genannten Produkts als gelbes Oel ergibt.

Analyse:
Berechnet für $C_{25}H_{35}N_3O$: C, 76,3; H, 9,0; N, 10,7.
Gefunden: C, 76,1; H, 8,9; N, 10,7.

Auf die gleiche Weise wird 5-Chlor-2-(2-hydroxy-3-dodecyl-5-methylphenyl)-2H-benztriazol hergestellt, jedoch unter Ersatz des oben verwendeten Benztriazols durch eine äquivalente Menge 5-Chlor-2-(2-hydroxy-5-methylphenyl)-2H-benztriazol.

Beispiel 7

2-(2-Hydroxy-3-tetrakosyl-5-methylphenyl)-2H-benztriazol

Gemäss der allgemeinen Arbeitsweise nach Beispiel 1 erhitzt man 112 g 2-(2-Hydroxy-5-methylphenyl)-2H-benztriazol, 33 ml Methansulfonsäure und 444 ml (2 Mol) eines Tetrakosengemischs (durch Dimerisierung von n-Dodecen hergestellt) 96 Stunden lang auf 160 °C. Dann kühlt man ab und isoliert das Produkt nach der in Beispiel 1 beschriebenen allgemeinen Arbeitsweise. Die überschüssigen Kohlenwasserstoffe werden durch Destillation bei 190 °C/0,04 mm entfernt. Das oben genannte Produkt erhält man in einer Ausbeute von 103,6 g als gelbe Flüssigkeit, welche noch inerte, verdünnende Kohlenwasserstoffe enthält, die durch Flash-Chromatographie unter Verwendung von Kieselgel und Heptan/Toluol 75/25 entfernt werden, was ein reines Produkt in einer Ausbeute von 15 g als gelbe Flüssigkeit ergibt.
Analyse:
Berechnet für $C_{37}H_{59}N_3O$: C, 79,1; H, 10,6; N, 7,5.
Gefunden: C, 79,3; H, 10,4; N, 8,1.

In den gemäss Beispielen 1-7 erhältlichen Produkten ist der Alkylrest in 3-Stellung, wie eingangs erklärt, eine statistische Mischung aus mindestens 3 Isomeren. Der Einfachkeit halber wurde dies nicht bei jedem Beispiel erwähnt.

Beispiel 8

Lichtstabilisierung eines Automobildecklacks

Eine für Automobildecklacke typische, duroplastische Acrylharzüberzugszusammensetzung wird mit 2 Gew.-% des in Beispiel 3 hergestellten Lichtschutzmittels formuliert. Die Ueberzugszusammensetzung wird auf eine Metalltafel aufgebracht und bei 130 °C zur Härtung des Harzes eingebrannt. Die überzogene Tafel wird dann einer Schnellbewitterungsprüfung unterzogen, bei der es sich um abwechselnde 8-Stundenperioden mit UV-Bestrahlung bei 70 °C und 4-Stundenperioden mit Kondensation (Regen) bei 50 °C pro Zyklus für insgesamt 980 Stunden handelt.

Die Werte für 20°-Glanz (ASTM D523 und D2457) und Bildschärfe (ASTM E 430) des Ueberzugs vor und nach der Bewitterung in der Schnellprüfung werden gemessen und die prozentuale Erhaltung des 20°-Glanzes und der Bildschärfe (D/I) berechnet. Als Kontrolle dient derselbe duroplastische Acrylharzüberzug ohne Stabilisator.

Die Ergebnisse sind unten in der Tabelle angegeben.

| Ueberzugseigenschaften nach 980 Stunden Aussetzung in der Schnellbewitterungsprüfung | | | |
|---|---|---|---|
| Duroplastisches Acrylharz* | Oberflächenrisse | % Erhaltung des 20°-Glanzes | % Erhaltung des D/I |
| Kontrolle (unstabilisiert) | ja | 42 | 38 |
| Mit 2 Gew.-% Stabilisator aus Beispiel 3 | nein | 72 | 97 |

*Die Grundlage für den duroplastischen Acryleinbrennlack ist ein Bindemittel aus 70% Acrylmonomeren wie Hydroxyäthylacrylat, Styrol, Acrylnitril, Butylacrylat und Acrylsäure sowie 30% Melaminharz.

Der stabilisierte Ueberzug zeigt weit überlegene Glanz- und D/I-Erhaltung als die unstabilisierte Kontrolle. Die stabilisierte Probe weist keine Anzeichen von Oberflächenspalten oder Rissen auf, was noch einmal die Wirksamkeit der vorliegenden Verbindungen als Lichtschutzmittel bestätigt.

Beispiel 9

Ein ölmodifizierter Urethanlack mit 2 Gew.-% des in Beispiel 3 hergestellten Stabilisators wird auf eine Aluminiumtafel aufgebracht und unter einem nach Süden gerichteten Winkel von 90° im Freien im südlichen New York für einen Zeitraum von 10,5 Monaten aufgestellt.

Der nach ASTM D 1925 gemessene Vergilbungsindex (YI) für die Probe wird vor und nach der Aussetzung gemessen. Die YI-Aenderung ist ein Mass für den Verfärbungsgrad des Urethanüberzugs während des Prüfungszeitraums. Je niedriger die YI-Aenderung, umso weniger ist die Probe verfärbt.

| Oelmodifizierter Urethanlack nach 10,5 Monaten Aussetzung im Freien | | |
|---|---|---|
| Probe | YI-Aenderung | % Glanz Erhaltung (20°) |
| Kontrolle (unstabilisiert ) | 14 | 95 |
| Probe mit 2 Gew.-% des Gemischs aus Beispiel 3 | -3,4 | 96 |

Die Glanzerhaltungswerte sind gleich, aber der die vorliegenden Benztriazole aus Beispiel 3 enthaltende Urethanlack vergilbt nicht, während die Kontrolle sich merklich verfärbte (vergilbte).

Beispiel 10

Schleierentwicklung in photographischen Zusammensetzungen

Es ist schwierig, die Verträglichkeit von Benztriazollichtschutzmitteln in photographischen Zusammensetzungen direkt zu beurteilen. Bei solche Stabilisatoren in photographischen Oelen enthaltenden Zusammensetzungen sind häufig längere Zeiten erforderlich, bevor Entmischung oder Schleier beobachtbar ist.

Eine wichtige Eigenschaft photographischer Zusammensetzungen, die mit solchen Verträglichkeitsgrössen in direkter Beziehung steht, ist die Schleierbildung. Zur Erzeugung klarer und scharfer photographischer Abbildungen ist es offensichtlich erforderlich, den Schleier auf ein Mindestmass zu verringern oder noch besser im wesentlichen zu beseitigen.

Gemäss der in U.S. Patent 4 383 863, Beispiel 5, beschriebenen Arbeitsweise wird eine UV-Schutzschicht in Gelatine hergestellt, die ein anionenaktives Netzmittel, einen Härter und den vorliegenden Stabilisator aus Beispiel 3 ohne Verwendung eines Lösungsmittels enthält.

Eine sehr feine Dispersion des vorliegenden Stabilisators in dieser Gelatinezusammensetzung wird durch Ultraschallmischen erzeugt, was eine UV-Schutzschicht ergibt, die klar und durchsichtig ist und keinen Schleier zeigt.

Beispiel 11

Schutz einer photographischen Purpurschicht

Eine UV-Filterschicht wird dadurch hergestellt, dass man 30 mg einer 1:1-Mischung aus 6-Chlor-2-[2'-hydroxy-3'-t-butyl-5'-$\beta$-(n-octyloxycarbonyl)äthylphenyl]-2H-bentriazol und 6-Chlor-2-[2'-hydroxy-3'-t-butyl-5'-$\beta$-(2"-äthylhexyloxycarbonyl)äthylphenyl]-2H-benztriazol zusammen mit 46 mg der Verbindung gemäss Beispiel 3 als UV-Absorber in 2 ml reinstem Essigester auflöst. 1 ml dieser Lösung wird mit 4 ml 6%iger Gelatinelösung, die 1,18 g/l des Natriumalkylnaphthalinsulfonattensids Nekal® BX (erhältlich von GAF Corp.) enthält, vermischt. Nach 3 Minuten Dispergieren mit Ultraschall vermischt man 2,5 ml der so entstandenen Emulsion mit 4,5 ml entionisiertem Wasser und 1 ml 0,7%iger Härterlösung und giesst die Emulsion von Hand auf einen 13 x 18 cm Bogen aus klarem Polyesterträger.

Auf ähnliche Weise wird eine Kontrollfilterschicht nach derselben Methode wie oben/aber ohne die beiden UV-Absorber hergestellt.

Eine Modellpurpurschicht wird dadurch hergestellt, dass man 0,19 mg Purpurkuppler der Formel

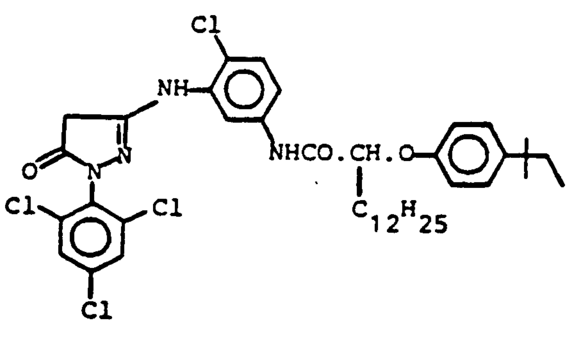

und 0,097 g Trikresylphosphat in 10 ml reinstem Essigester auflöst. 1 ml dieser Lösung wird mit 9 ml einer 2,3%igen Gelatinelösung, die 0,43 g/l Nekal® BX enthält, vermischt. Nach 3 Minuten Dispergieren mit Ultraschall vermischt man 5 ml der so erhaltenen Emulsion mit 2 ml einer photographischen Silberbromidemulsion, die 0,012% Ag und 0,15 g Gelatine sowie 1 ml einer 0,7%igen Lösung des Härters der Formel

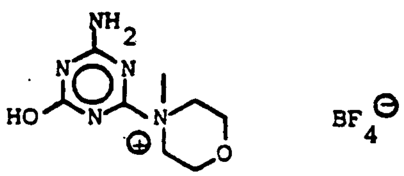

enthält. Das so erhaltene Gemisch wird von Hand auf einen 13 x 18 cm Bogen aus photographischem Papierträger gegossen. Nach 1 Woche langer Trocknung und Härtung bei Raumtemperatur wird das Papier durch eine Stufentablette hindurch belichtet und unter Verwendung von Verarbeitungschemikalien Kodak Ektaprint 2 entwickelt, was eine Purpurabbildung der Stufentablette ergibt.

Die Stufentablettenpurpurabbildungen werden in einem Atlas-Weatherometer bei 88 klux dem Licht einer Xenonlampe mit zwei Borsilikatfiltern und einem zusätzlichen Filter aus 8 mm Fensterglas, das um die Lampe herum angeordnet ist, um Sonnenlicht in einem Zimmer zu simulieren, ausgesetzt. Die UV-Filter und die Kontrollfilter werden durch Einfügen zwischen zwei dünnen Glasplatten auf einem Metallprobenhalter über den Purpurprüfungsproben gehalten. Die Purpurfarbstoffverluste bei der Belichtung werden für das UV-Filter und das Kontrollfilter bei einer Dichte von 1,00 unter Verwendung eines mit Status-A-Filtern im Grünkanal ausgerüsteten Macbeth TR 924-Dichtemessgeräts bestimmt. Die Zunahme der Vergilbung der beiden Proben wird auf ähnliche Weise unter Verwendung desselben Dichtemessgeräts gemessen, indem man die Zunahme der Blaudichte an den Nichtbildstellen der Probe verfolgt.

Die Ergebnisse in der Tabelle zeigen deutlich die Ueberlegenheit des die UV-Absorber enthaltenden Filters über das Kontrollfilter beim Schutz des Purpurfarbstoffs gegen Lichtausbleichung und des Purpurkupplers gegen Vergilbung.

Tabelle

| | Verlust an Purpurfarbstoff $D_G$ = 1,00 (in %) bei Belichtung | | Zunahme der Vergilbung ($100\Delta D_B$) bei Belichtung | |
|---|---|---|---|---|
| | nach 15kJ/cm$^2$ | nach 30 kJ/cm$^2$ | nach 15 kJ/cm$^2$ | nach 30 kJ/cm$^2$ |
| mit UV Filter | 49 | 79 | 10 | 17 |
| mit Kontrollfilter | 66 | 81 | 24 | 29 |

**Patentansprüche**

1. Flüssige oder nicht-kristalline Gemische von Benztriazolen, die im wesentlichen aus Verbindungen der Formel

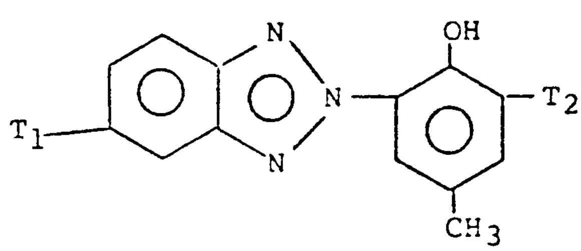

(I)

bestehen, worin $T_1$ Wasserstoff oder Chlor und $T_2$ ein statistisches Gemisch aus mindestens drei isomeren verzweigten sekundären Alkylgruppen mit je 8 bis 30 Kohlenstoffatomen der Formel

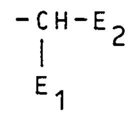

worin $E_1$ für geradkettiges Alkyl mit 1 bis 14 Kohlenstoffatomen und $E_2$ für geradkettiges Alkyl mit 4 bis 15 Kohlenstoffatomen stehen, wobei die Gesamtzahl Kohlenstoffatome in $E_1$ plus $E_2$ 7 bis 29 beträgt, darstellen, erhältlich durch Alkylierung einer Verbindung der Formel

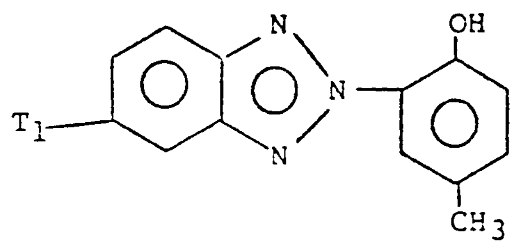

mit einem geradkettigen Alken mit 8 bis 30 Kohlenstoffatomen in Gegenwart eines sauren Katalysators und bei einer Temperatur von 100-200 °C.

**2.** Gemische nach Anspruch 1, dadurch gekennzeichnet, dass $T_1$ für Wasserstoff steht.

**3.** Gemische nach Anspruch 1, dadurch gekennzeichnet, dass $T_2$ für Alkyl mit 8 bis 16 Kohlenstoffatomen steht.

**4.** Gemische nach Anspruch 3, dadurch gekennzeichnet, dass $T_2$ für Alkyl mit 10 bis 12 Kohlenstoffatomen steht.

**5.** Flüssige oder nicht-kristalline Gemische von Benztriazolen, die im wesentlichen aus Verbindungen der Formel

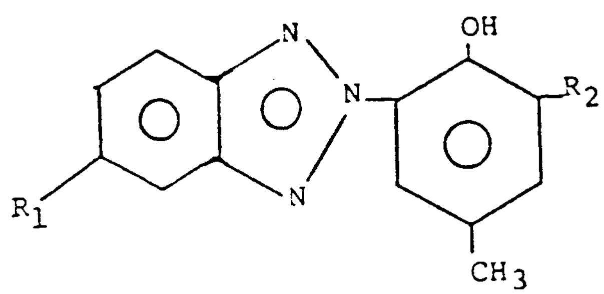

(II)

bestehen, worin $R_1$ Wasserstoff oder Chlor und $R_2$ ein statistisches Gemisch aus mindestens drei isomeren verzweigten Alkylgruppen mit je 8 bis 30 Kohlenstoffatomen und mit einer Mehrzahl Alkylverzweigungen entlang der Hauptalkylkette darstellen, erhältlich durch Alkylierung eines Benztriazols der Formel

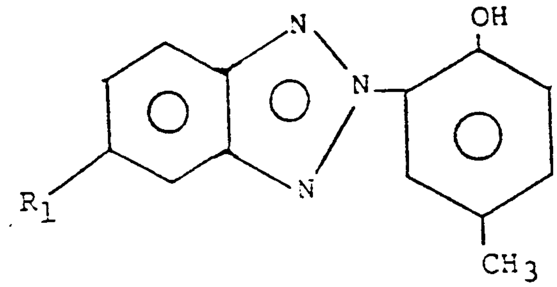

mit einem verzweigten Alken mit 8 bis 30 Kohlenstoffatomen in Gegenwart eines sauren Katalysators und bei einer Temperatur von 100-200 °C.

**6.** Gemische nach Anspruch 5, dadurch gekennzeichnet, dass $R_1$ für Wasserstoff steht.

**7.** Gemische nach Anspruch 5, dadurch gekennzeichnet, dass $R_2$ für Alkyl mit 8 bis 16 Kohlenstoffatomen steht.

**8.** Gemische nach Anspruch 7, dadurch gekennzeichnet, dass $R_2$ für Alkyl mit 9 bis 12 Kohlenstoffatomen steht.

**9.** Verfahren zur Herstellung von flüssigen oder nichtkristallinen Gemischen von Benztriazolen, die im wesentlichen aus Verbindungen der Formel

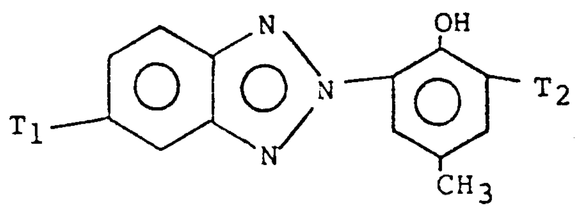

(I)

bestehen, worin $T_1$ Wasserstoff oder Chlor und $T_2$ ein statistisches Gemisch aus mindestens drei isomeren verzweigten sekundären Alkylgruppen mit je 8 bis 30 Kohlenstoffatomen der Formel

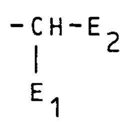

worin $E_1$ für geradkettiges Alkyl mit 1 bis 14 Kohlenstoffatomen und $E_2$ für geradkettiges Alkyl mit 4 bis 15 Kohlenstoffatomen stehen, wobei die Gesamtzahl Kohlenstoffatome in $E_1$ plus $E_2$ 7 bis 29 beträgt, darstellen, dadurch gekennzeichnet, dass man ein Benztriazol der Formel

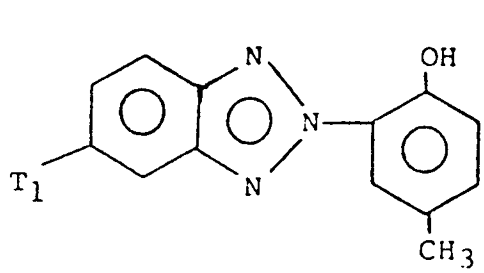

worin $T_1$ Wasserstoff oder Chlor darstellt, in Gegenwart eines sauren Katalysators bei einer Temperatur von 100 bis 200 °C mit einem geradkettigen Alken mit 8 bis 30 Kohlenstoffatomen alkyliert.

**10.** Verfahren zur Herstellung flüssiger oder nicht-kristalliner Gemische von Benztriazolen, welche im wesentlichen aus Verbindungen der Formel

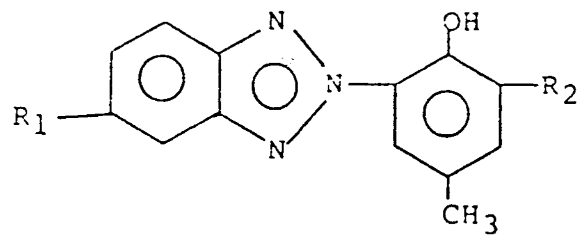

bestehen, worin $R_1$ Wasserstoff oder Chlor und $R_2$ ein statistisches Gemisch aus mindestens drei isomeren verzweigten Alkylgruppen mit je 8 bis 30 Kohlenstoffatomen und mit einer Mehrzahl Alkylverzweigungen entlang der Hauptalkylkette darstellen, dadurch gekennzeichnet, dass man ein Benztriozol der Formel

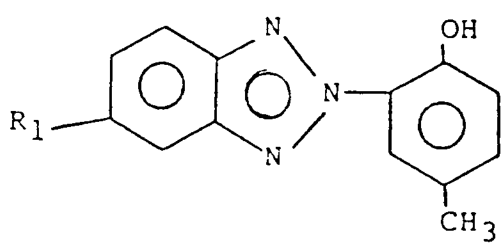

worin $R_1$ Wasserstoff oder Chlor darstellt, in Gegenwart eines sauren Katalysators bei einer Temperatur von 100 bis 200 °C mit einem verzweigten Alken mit 8 bis 30 Kohlenstoffatomen alkyliert.

**11.** Verfahren nach Anspruch 9 oder 10, dadurch gekennzeichnet, dass die Temperatur 140 bis 170 °C, vorzugsweise 160-165 °C, beträgt.

**12.** Verfahren nach Anspruch 9 oder 10, dadurch gekennzeichnet, dass das Aequivalenzverhältnis von Alken zu 2H-Benztriazol 1:1 bis 6:1, vorzugsweise 3,5:1 bis 4,5:1, beträgt.

**13.** Verfahren nach Anspruch 9 oder 10, dadurch gekennzeichnet, dass der saure Katalysator aus der aliphatische, aromatische und substituierte aromatische Sulfonsäuren, Schwefelsäure, Phosphorsäure, saure Tone und saure Molekularsiebe umfassenden Gruppe ausgewählt ist.

**14.** Verfahren nach Anspruch 9 oder 10, dadurch gekennzeichnet, dass als saurer Katalysator eine aliphatische Sulfonsäure, vorzugsweise Methansulfonsäure, verwendet wird.

**15.** Verfahren nach Anspruch 9 oder 10, dadurch gekennzeichnet,dass sich die Aequivalente saurer Katalysator zu den Aequivalenten Benztriazol wie 0,2 zu 3, vorzugsweise wie 0,5 zu 1, verhalten.

**16.** Stabilisierte Zusammensetzungen, dadurch gekennzeichnet, dass sie aus (a) einem organischen Material und (b) einer wirksamen Menge eines Gemischs von Stabilisatoren der Formel I nach Anspruch 1 oder der Formel II nach Anspruch 5 bestehen.

**17.** Stabilisierte Zusammensetzungen nach Anspruch 16, dadurch gekennzeichnet, dass als organisches Material (a) ein organisches Polymer vorliegt.

**18.** Stabilisierte Zusammensetzungen nach Anspruch 17, dadurch gekennzeichnet, dass als organisches Polymer ein Polyolefin, ein Styrolpolymer, ein Polyacrylat, ein Polyamid, ein Polyurethan, ein halogenhaltiges Vinylpolymer, ein Alkydharz, ein duroplastisches Acrylharz oder ein Epoxyharz vorliegt.

**19.** Stabilisierte Zusammensetzungen nach Anspruch 16, dadurch gekennzeichnet, dass das organische Material in Form photographischen Materials oder als Teil eines photographischen Elements vorliegt.

**20.** Verwendung von Gemischen der Formel I nach Anspruch 1 oder der Formel II nach Anspruch 5 zum Stabilisieren von organischen polymeren Materialien.

**21.** Verwendung nach Anspruch 20 zum Stabilisieren von Polyolefinen, Styrolpolymeren, Polyacrylaten, Polyamiden, Polyurethanen, halogenhaltigen Vinylpolymeren, Alkydharzen, duroplastischen Acrylharzen oder Epoxyharzen.

**22.** Verwendung nach Anspruch 20 zum Stabilisieren von photographischem organischem Material.

**Claims**

**1.** A liquid or non-crystalline mixture of benzotriazoles which consists essentially of compounds of the formula

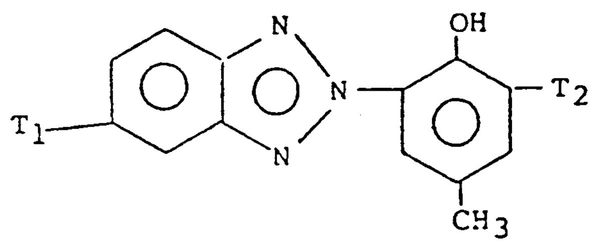

(I)

in which $T_1$ is hydrogen or chlorine and $T_2$ is a random statistical mixture of at least three isomeric branched secondary alkyl groups each having 8 to 30 carbon atoms and of the formula

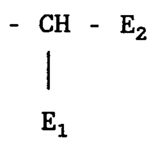

in which $E_1$ is straight-chain alkyl of 1 to 14 carbon atoms and $E_2$ is straight-chain alkyl of 4 to 15 carbon atoms, where the total number of carbon atoms in $E_1$ plus $E_2$ is 7 to 29, obtainable by alkylating a compound of the formula

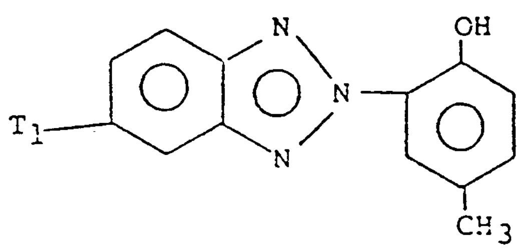

with a straight-chain alkene of 8 to 30 carbon atoms in the presence of an acidic catalyst and at a temperature of 100-200 ° C.

**2.** A mixture according to claim 1, wherein $T_1$ is hydrogen.

**3.** A mixture according to claim 1, wherein $T_2$ is alkyl of 8 to 16 carbon atoms.

**4.** A mixture according to claim 3, wherein $T_2$ is alkyl of 10 to 12 carbon atoms.

**5.** A liquid or non-crystalline mixture of benzotriazoles which consists essentially of compounds of the formula

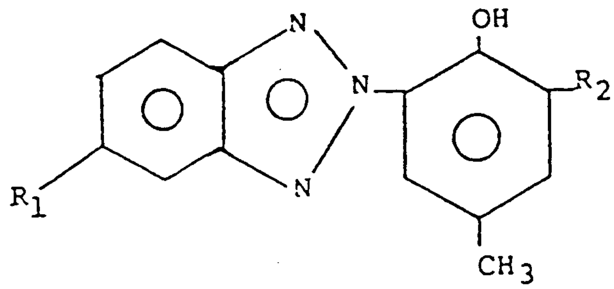

(II)

in which $R_1$ is hydrogen or chlorine and $R_2$ is a random statistical mixture of at least three isomeric branched alkyl groups each having 8 to 30 carbon atoms and having a multiplicity of alkyl branches along the main alkyl chain, obtainable by alkylating a benzotriazole of the formula

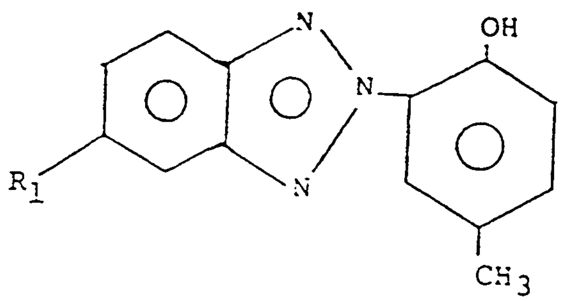

with a branched alkene of 8 to 30 carbon atoms in the presence of an acidic catalyst and at a temperature of 100-200 ° C.

**6.** A mixture according to claim 5, wherein $R_1$ is hydrogen.

**7.** A mixture according to claim 5, wherein $R_2$ is alkyl of 8 to 16 carbon atoms.

**8.** A mixture according to claim 7, wherein $R_2$ is alkyl of 9 to 12 carbon atoms.

**9.** A process for preparing a liquid or non-crystalline mixture of benzotriazoles which consists essentially of compounds of the formula

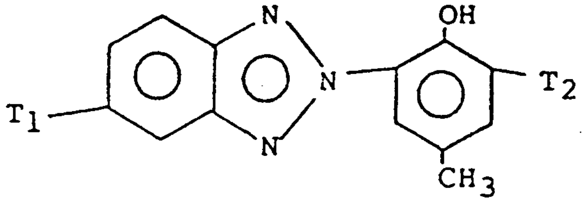

(I)

in which $T_1$ is hydrogen or chlorine and $T_2$ is a random statistical mixture of at least three isomeric branched secondary all groups each having 8 to 30 carbon atoms and of the formula

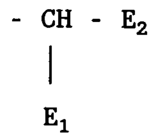

in which $E_1$ is straight-chain alkyl of 1 to 14 carbon atoms and $E_2$ is straight-chain alkyl of 4 to 15 carbon atoms where the total number of carbon atoms in $E_1$ plus $E_2$ is 7 to 29, which comprises alkylating a benzotriazole of the formula

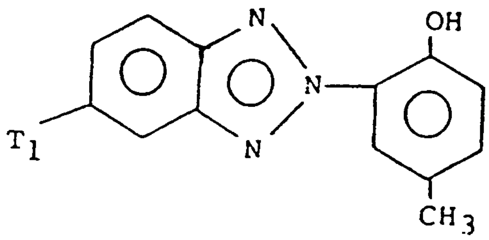

in which $T_1$ is hydrogen or chlorine in the presence of an acidic catalyst at a temperature of 100 to 200°C with a straight-chain alkene of 8 to 30 carbon atoms.

**10.** A process for preparing a liquid or non-crystalline mixture of benzotriazoles which consists essentially of compounds of the formula

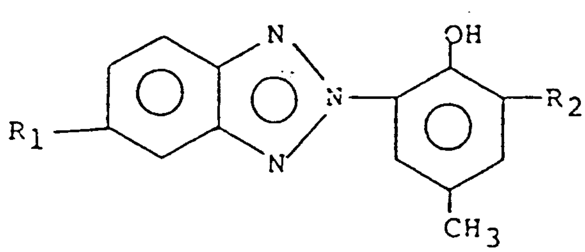

in which $R_1$ is hydrogen or chlorine and $R_2$ is a random statistical mixture of at least three isomeric branched alkyl groups each having 8 to 30 carbon atoms and having a multiplicity of alkyl branches along the main alkyl chain, which comprises alkylating a benzotriazole of the formula

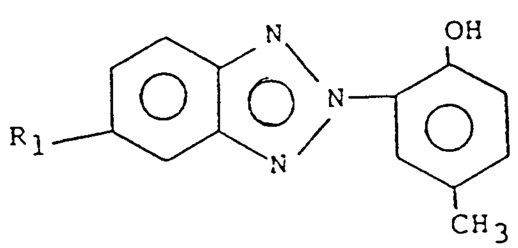

in which $R_1$ is hydrogen or chlorine in the presence of an acidic catalyst at a temperature of 100 to 200 ° C with a branched alkene of 8 to 30 carbon atoms.

**11.** A process according to claim 9 or 10, wherein the temperature is 140 to 170 ° C, preferably 160-165 ° C.

**12.** A process according to claim 9 or 10, wherein the equivalent ratio of alkene to 2H-benzotriazole is from 1:1 to 6:1, preferably from 3.5:1 to 4.5:1.

**13.** A process according to claim 9 or 10, wherein the acidic catalyst is selected from the group consisting of aliphatic, aromatic and substituted aromatic sulfonic acids, sulfuric acid, phosphoric acid, acidic clays and acidic molecular sieves.

**14.** A process according to claim 9 or 10, wherein the acidic catalyst used is an aliphatic sulfonic acid, preferably methanesulfonic acid.

**15.** A process according to claim 9 or 10, wherein the ratio of equivalents of acidic catalyst to equivalents of benzotriazole is 0.2 to 3, preferably 0.5 to 1.

**16.** A stabilized composition which comprises
(a) an organic material, and (b) an effective amount of a mixture of stabilizers of the formula I according to claim 1 or of the formula II according to claim 5.

**17.** A stabilized composition according to claim 16, wherein the organic material (a) is an organic polymer.

**18.** A stabilized composition according to claim 17, wherein the organic polymer is a polyolefin, a styrene polymer, a polyacrylate, a polyamide, a polyurethane, a halogen-containing vinyl polymer, an alkyd resin, a thermosetting acrylic resin or an epoxy resin.

**19.** A stabilized composition according to claim 16, wherein the organic material is in the form of photographic material or part of a photographic element.

**20.** The use of a mixture of the formula I according to claim 1 or of the formula II according to claim 5 for stabilizing organic polymeric materials.

**21.** The use according to claim 20 for stabilizing polyolefins, styrene polymers, polyacrylates, polyamides, polyurethanes, halogen-containing vinyl polymers, alkyd resins, thermosetting acrylic resins or epoxy resins.

**22.** The use according to claim 20 for stabilizing photographic organic material.

**Revendications**

**1.** Mélanges de benzotriazoles liquides ou non cristallisés qui sont essentiellement constitués de composés répondant à la formule I :

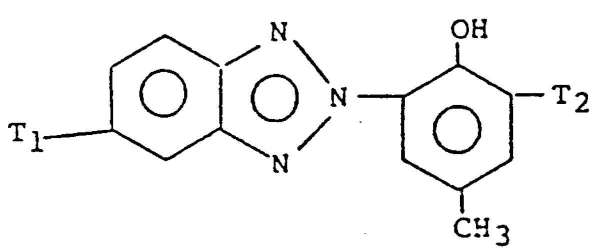

(I)

dans laquelle $T_1$ représente l'hydrogène ou le chlore et $T_2$ un mélange statistique constitué d'au moins trois radicaux alkyles secondaires ramifiés isomères, qui contiennent chacun de 8 à 30 atomes de carbone et répondent à la formule :

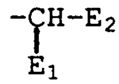

dans laquelle $E_1$ représente un alkyle linéaire contenant de 1 à 14 atomes de carbone et $E_2$ un alkyle linéaire contenant de 4 à 15 atomes de carbone, le nombre total des atomes de carbone contenus dans l'ensemble ($E_1$, $E_2$) étant compris entre 7 et 29, mélanges que l'on peut obtenir en alkylant un composé de formule :

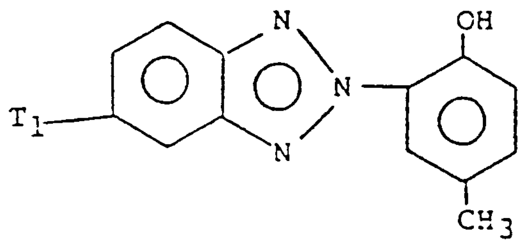

avec un alcène linéaire contenant de 8 à 30 atomes de carbone, en présence d'un catalyseur acide et à une température de 100 à 200 °C.

**2.** Mélanges selon la revendication 1 caractérisés en ce que $T_1$ représente l'hydrogène.

**3.** Mélanges selon la revendication 1 caractérisés en ce que $T_2$ représente un alkyle contenant de 8 à 16 atomes de carbone.

**4.** Mélanges selon la revendication 3 caractérisés en ce que $T_2$ représente un alkyle contenant de 10 à 12 atomes de carbone.

**5.** Mélanges liquides ou non cristallisés de benzotriazoles qui sont essentiellement constitués de composés répondant à la formule II :

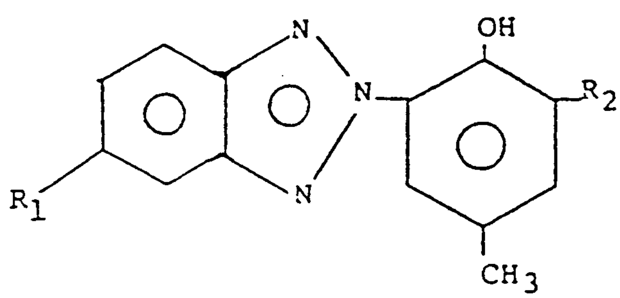

(II)

dans laquelle $R_1$ représente l'hydrogène ou le chlore et $R_2$ un mélange statistique d'au moins trois alkyles ramifiés isomères qui contiennent chacun de 8 à 30 atomes de carbone et qui portent plusieurs ramifications alkyles le long de la chaîne alkyle principale, mélanges que l'on peut obtenir en alkylant un benzotriazole de formule :

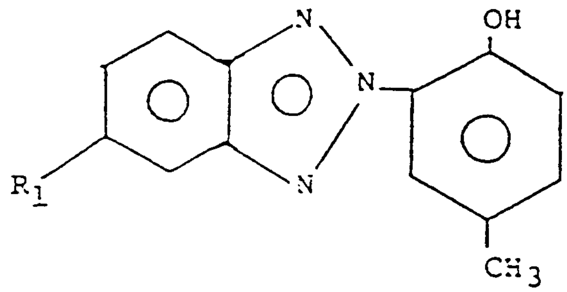

avec un alcène ramifié contenant de 8 à 30 atomes de carbone, en présence d'un catalyseur acide et à une température de 100 à 200 ° C.

**6.** Mélanges selon la revendication 5 caractérisés en ce que $R_1$ représente l'hydrogène.

**7.** Mélanges selon la revendication 5 caractérisés en ce que $R_2$ représente un alkyle contenant de 8 à 16 atomes de carbone.

**8.** Mélanges selon la revendication 7 caractérisés en ce que $R_2$ représente un alkyle contenant de 9 à 12 atomes de carbone.

**9.** Procédé pour préparer des mélanges liquides ou non cristallisés de benzotriazoles essentiellement constitués de composés répondant à la formule I :

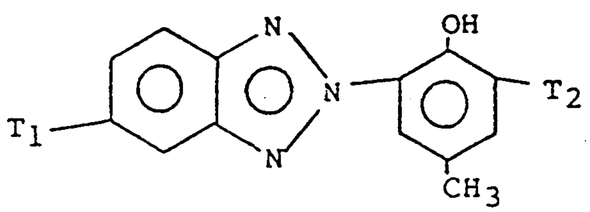

(I)

dans laquelle $T_1$ représente l'hydrogène ou le chlore et $T_2$ un mélange statistique d'au moins trois alkyles secondaires ramifiés isomères qui contiennent chacun de 8 à 30 atomes de carbone et répondent à la formule :

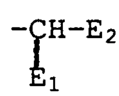

dans laquelle $E_1$ représente un alkyle linéaire contenant de 1 à 14 atomes de carbone et $E_2$ un alkyle linéaire contenant de 4 à 15 atomes de carbone, le nombre total des atomes de carbone contenus dans l'ensemble ($E_1$, $E_2$) étant de 7 à 29, procédé caractérisé en ce qu'on alkyle un benzotriazole répondant à la formule :

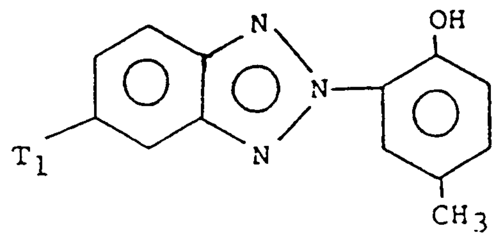

dans laquelle $T_1$ représente l'hydrogène ou le chlore, en présence d'un catalyseur acide, à une température de 100 à 200 ° C, avec un alcène linéaire contenant de 8 à 30 atomes de carbone.

**10.** Procédé pour préparer des mélanges liquides ou non cristallisés de benzotriazoles essentiellement constitués de composés répondant à la formule :

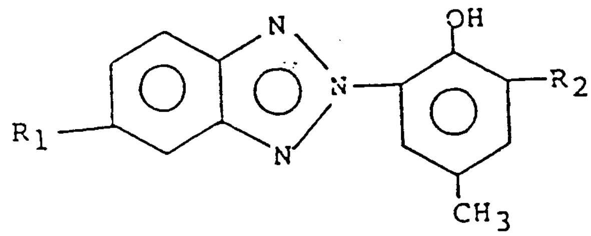

dans laquelle $R_1$ représente l'hydrogène ou le chlore et $R_2$ un mélange statistique d'au moins trois alkyles ramifiés isomères qui contiennent chacun de 8 à 30 atomes de carbone et qui portent plusieurs ramifications alkyles le long de la chaîne alkyle principale, procédé caractérisé en ce qu'on alkyle un benzotriazole répondant à la formule :

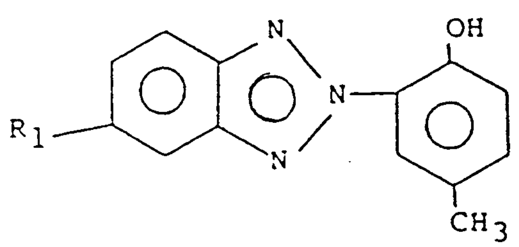

dans laquelle $R_1$ représente l'hydrogène ou le chlore, en présence d'un catalyseur acide, à une température de 100 à 200 ° C, avec un alcène ramifié contenant de 8 à 30 atomes de carbone.

**11.** Procédé selon l'une des revendications 9 et 10, caractérisé en ce que la température est comprise entre 140 et 170 ° C, de préférence entre 160 et 165 ° C.

**12.** Procédé selon l'une des revendications 9 et 10, caractérisé en ce que le rapport, en équivalents, de l'alcène au 2H-benzotriazole est compris entre 1:1 et 6:1, de préférence entre 3,5:1 et 4,5:1.

**13.** Procédé selon l'une des revendications 9 et 10, caractérisé en ce que le catalyseur acide est choisi dans l'ensemble constitué par les acides sulfoniques aliphatiques, aromatiques et aromatiques substitués, l'acide sulfurique, l'acide phosphorique, les argiles acides et les tamis moléculaires acides.

**14.** Procédé selon l'une des revendications 9 et 10, caractérisé en ce qu'on utilise, comme catalyseur acide, un acide sulfonique aliphatique, de préférence l'acide méthanesulfonique.

**15.** Procédé selon l'une des revendications 9 et 10, caractérisé en ce que le rapport, en équivalents, entre

le catalyseur acide et le benzotriazole est compris entre 0,2 et 3, de préférence entre 0,5 et 1.

**16.** Compositions stabilisées caractérisées en ce qu'elles sont constituées (a) d'une matière organique et (b) d'une quantité efficace d'un mélange de stabilisants de formule I selon la revendication 1 ou de formule II selon la revendication 5.

**17.** Compositions stabilisées selon la revendication 16, caractérisées en ce que la matière organique (a) est un polymère organique.

**18.** Compositions stabilisées selon la revendication 17, caractérisées en ce que le polymère organique est une polyoléfine, un polymère du styrène, un polyacrylate, un polyamide, un polyuréthanne, un polymère vinylique halogéné, une résine alkyde, une résine acrylique thermodurcissable ou une résine époxydique.

**19.** Compositions stabilisées selon la revendication 16, caractérisées en ce que la matière organique est sous la forme d'une matière photographique ou d'une partie d'un élément photographique.

**20.** Application de mélanges de formule I selon la revendication 1 ou de formule II selon la revendication 5 pour la stabilisation de matières polymères organiques.

**21.** Application selon la revendication 20 pour la stabilisation de polyoléfines, de polymères du styrène, de polyacrylates, de polyamides, de polyuréthannes, de polymères vinyliques halogénés, de résines alkydes, de résines acryliques thermodurcissables ou de résines époxydiques.

**22.** Application selon la revendication 20 pour la stabilisation d'une matière organique photographique.